(19) Europäisches Patentamt
European Patent Office
Office européen des brevets

(11) EP 4 684 640 A1

(12) EUROPEAN PATENT APPLICATION

(43) Date of publication:
**28.01.2026 Bulletin 2026/05**

(21) Application number: **24190327.7**

(22) Date of filing: **23.07.2024**

(51) International Patent Classification (IPC):
***A01N 43/54*** $^{(2006.01)}$ ***A01N 43/84*** $^{(2006.01)}$
***A01N 47/44*** $^{(2006.01)}$ ***A01P 21/00*** $^{(2006.01)}$
***C07D 239/95*** $^{(2006.01)}$ ***C07F 7/02*** $^{(2006.01)}$

(52) Cooperative Patent Classification (CPC):
**A01P 21/00; A01N 43/54; A01N 43/84;**
**A01N 47/44; C07D 239/95;** C07F 7/0812

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Leibniz-Institut für Pflanzenbiochemie (IPB)**
**Stiftung des öffentlichen Rechts**
**06120 Halle (Saale) (DE)**

(72) Inventors:
- **Berger, Robert**
  **06800 Raguhn-Jeßnitz (DE)**
- **Wessjohann, Ludger**
  **06120 Halle (Saale) (DE)**
- **Gonzalez Ceballos, Leonardo**
  **06110 Halle (Saale) (DE)**
- **Heym, Peter Paul**
  **04509 Delitzsch (DE)**

(74) Representative: **Müller-Boré & Partner**
**Patentanwälte PartG mbB**
**Friedenheimer Brücke 21**
**80639 München (DE)**

(54) **QUINAZOLINONES AS PHYTOEFFECTORS**

(57) The present invention relates to the use of specific quinazolinones as phytoeffectors, e.g. for increasing plant growth, as well as to respective compounds as such.

EP 4 684 640 A1

## Description

The present invention relates to the use of specific quinazolinones as phytoeffectors, e.g. for increasing plant growth, as well as to respective compounds as such.

"Plant stress" in general is understood to describe environmental conditions that hinder a plants' development - growth, productivity, reproduction and survival - through induction of adverse physiological reactions. Throughout their entire life cycle, plants are exposed to a variety of stressors, which can primarily be divided into two classes: organisms that infect or feed on a plant are regarded biotic stressors, while all climatic, physical and chemical influences in a broader sense are considered abiotic stressors. Naturally, all plants are target to different stresses - however, a special interest in understanding, mitigating and even preventing plant stress exists in an agricultural environment. With the ever-increasing demand for crop plants used as food and feed - despite diverse industrial applications, e. g. in bioethanol production - the humanitarian and economic impact of substantial crop losses steadily grows as well. While 38 % of the global land area are already used for crop production and ever advancing agricultural technology and improved breeding enhance crop yields, the number of undernourished people is increasing anew since 2014, reaching about 735 million between 2021 and 2023. This counter-intuitive development is not least due to more extreme climatic variability and the substantial gain in global temperature, leading to severe droughts.

Drought as leading cause for crop losses is merely a facet of a set of environmental conditions that can be summarized as "water stress". This stress situation is characterized either by a limited availability of water or a restricted ability of the plant to uptake water that is present, the latter known as "physiological drought". The main causes for such deficiencies include (a) insufficient water activity within the soil (which describes the availability of water rather than its concentration) due to drought, high salinity or frost, (b) increased transpiration of the plant due to elevated temperatures, or (c) floods. Therefore, "drought" and "water stress" are used as synonyms whenever no clear distinction between drought and e. g. high salinity is possible or necessary.

Depending on its constitution, each plant has an individually preferred range of water activity in the soil - still, under sufficiently prolonged withdrawal of water, even the most specialized plant will dehydrate and eventually die. Dehydration and death are accompanied by different physiological signs of decay, which originate from complex changes in the most central molecular metabolic pathways including photosynthesis, protein synthesis and redox metabolism, as well as cell membrane integrity. The effects on the molecular level, including sophisticated signaling pathways, have been extensively investigated and reviewed.

One of the first facultative responses to water stress is an osmotic adjustment through accumulation of compounds, that exhibit a strong osmotic activity but at the same time do not negatively affect cellular metabolism. Such compounds are known as osmolytes. In order to guarantee water supply, the plant needs to actively lower its intracellular water potential to a level below the water potential of the surrounding soil. Otherwise, water uptake from the soil and influx into the cell is physically impossible and the plant cannot maintain its turgor, which is the osmotic pressure that is created through physically bound water inside a cell in comparison to its extracellular matrix. Following a loss in turgor, the cell size decreases which results in an inhibition of plant growth and is accompanied by wilting of leaves. The degree to which an accumulation of osmolytes like proline or glycine betaine can mitigate an unfavorable discrepancy in water potential strongly depends on the plant species, with so-called xerophytes being best adapted to arid conditions.

The impact of water deficiency onto the photosynthesis - which constitutes the main anabolic pathway in plants - is especially strong. Water is of central importance as electron donor for the reduction of $NADP^+$ to NADPH in the course of oxygenic photosynthesis. As result of a deficiency in water, and consequently NADPH, the reduction of 1,3-bisphosphoglycerate is hindered, which ultimately inhibits the biosynthesis of glucose and leads to an inhibition in growth. Consequently, another early response to water stress is the rapid closure of leaf stomata. The plants' plausible reaction to mitigate the effects of a deficiency in water uptake is the limitation of additional loss in water by evaporation. This evaporation takes place whenever the leaf stomata open to enable the assimilation of carbon dioxide, which is the second necessary substrate for carbohydrate synthesis, besides water. On the molecular level, regulation of the stomatal state, and thus transpiration, is controlled by the phytohormone abscisic acid (ABA). In case of a water deficiency, the signaling pathway leads to an increase in ABA concentration, which in turn triggers stomatal closure. Alas, with the stomata closed, exchange of both water vapour and carbon dioxide with the environment is practically impossible. Concludingly, uptake of one of the main substrates of photosynthesis is blocked, which afflicts the dark reaction and thus photosynthesis overall.

A third effect of water stress is the generation of reactive oxygen species (ROS). The described disturbance of photosynthesis leads to a misbalance between formation and depletion of redox equivalents in favor of oxidizing agents. These excess oxidants are responsible for the formation of ROS like singlet oxygen $^1O_2$, hyperoxide anions $O^{e}{}_2^{-}$, hydrogen peroxide $H_2O_2$ and a variety of radicals such as the hydroxyl radical, which are well-known to pose a serious threat to biologically important structures like membranes and proteins. For this reason, upregulation of the plants' ROS detoxification mechanisms is part of the stress response: Enzymes such as catalases, peroxidases and superoxide dismutases allow direct chemical transformation of reactive species, while other compounds, namely ascorbic acid and glutathione, act as ROS scavengers, which are recycled from their oxidized state by specialized enzymes.

Plant growth regulators (PGRs) in general are understood as naturally occurring or synthetic compounds that influence the development of plants through stimulation or inhibition of parts of the growth regulatory system, mostly involving the main plant hormones auxins, gibberellins, cytokinins, abscisins, strigolactones, and ethylene. Observable effects include the promotion, delay and prevention of growth, rooting, flowering, fruiting and aging. Thereby, PGRs mostly affect signaling pathways, which are also known to be important in plant stress response and tolerance. Thus, stress tolerance-inducing agents are hypothesized to exhibit plant growth- regulating effects in general, even in absence of abiotic stressors.

Among the marketed synthetic compounds with stress-protecting effects, the class of imidazole-and triazole-based fungicides takes a central position and shall therefore be exemplified here: Originally designed and developed for the treatment of fungal diseases in plants, several compounds exhibit additional growth-regulating and stress tolerance-inducing activity. Tri-adimefon for instance is not only an effective inhibitor of ergosterol biosynthesis in fungi but also interferes with the isoprenoid pathway in plants, thus affecting the biosynthesis of gibberellins, cytokinins and abscisic acid. It has been demonstrated that the triazole derivative effectively increases stomatal resistance in tomato plants, thus reducing transpiration under water stress, which led to an increase of 20 - 30 % in fruit fresh weight compared to the controls. It could further be shown that the effect of salt stress on physiological markers in *Withania somnnifera* (winter cherry), namely germination rate and chlorophyll content, as well as molecular antioxidant markers (ascorbic acid, glutathione and the enzymes superoxide dismutase and peroxidase) could be mitigated by treatment with triadimefon. Additional investigations revealed that the imidazole-based fungicide ketoconazole induces drought stress tolerance in *Catharanthus resets* (rosy periwinkle) through promotion of the antioxidant potential, both on protein level (upregulation of e. g. superoxide dismutase and catalase) as well as on the level of small molecule ROS scavengers. A most recent example of salt stress reduction in an agriculturally important crop, wheat (*Triticum aestivum* L.), was reported. By treatment of seeds ("priming") with 6-benzylaminopurine, a synthetic cytokinin, considerable stress tolerance was achieved.

Eventually, the class of neonicotinoids is worth mentioning due to their importance in industrial agriculture and the controversy linked to their application, among which imidacloprid is of central relevance. Released for industrial application as pest control in 1994 by the Bayer AG (Bayer CropScience), the agent rose to become the most widely sold and used insecticide in the world, before beneficial side effects in relation to stress tolerance and enhanced crop yield were reported. Initially to be considered an off-label use, the application as stress tolerance-inducing agent was commercialized in 2006 as "Confidor ® Stress Shield Inside", a product proclaiming both activity as pest control, as well as drought stress tolerance-enhancer. While the mode of action for the induction of stress tolerance has not been elucidated conclusively, 6-chloronicotinic acid as major degradation product of imidacloprid was proposed to be the active principle, effecting a reduction in plant poly(ADP-ribose) polymerase (PARP) activity.

Accordingly, the technical problem underlying the present invention is the provision of compounds acting as phytoeffectors, in particular compounds having plant stress-protecting effects.

The solution to the above technical problem is achieved by the embodiments characterized in the claims.

In particular, in a first aspect, the present invention relates to the use of a compound according to Formula (I) as a phytoeffector:

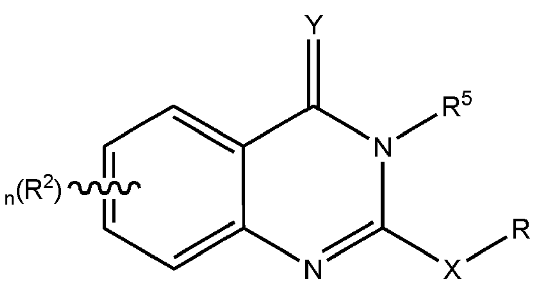

(I)

wherein

Y is O, $N\text{-}NR^3_2$, $N\text{-}OR^3$, or $NR^3$,

X is $NR^4$, S, SO, $SO_2$, O, or $CH_2$,

R is H, alkyl, cycloalkyl, unsubstituted or substituted aryl, or unsubstituted or substituted alkaryl; or, if X stands for $CH_2$, then, R can also be alkoxy, unsubstituted or substituted oxyaryl, or morpholinyl,

$R^2$ is H, halogen, nitro, unsubstituted or substituted amino, alkyl, alkoxy, unsubstituted or substituted alkynyl or unsubstituted or substituted aryl; or $R^2$ stands for one or more annellated aromatic rings to form a naphthalene, anthracene, or phenanthrene moiety,

$R^3$ is H, alkyl, alkaryl, unsubstituted or substituted heteroaryl or unsubstituted or substituted aryl, and

$R^4$ is H, alkyl, alkaryl, unsubstituted or substituted heteroaryl or unsubstituted or substituted aryl,

$R^5$ is H, alkyl, alkaryl, unsubstituted or substituted heteroaryl or unsubstituted or substituted aryl, and

n is 0, 1, 2, 3, or 4,

where the aforementioned substituent(s) can be selected from the group consisting of halogen, alkyl, alkoxy, aryloxy, trialkylsilyl, nitro, unsubstituted or substituted amino including aminoheterocycles such as e.g. morpholino.

In the compound according to Formula (I) of the present invention, it is preferred that Y is O. Further, it is preferred that X is NH. Furthermore, it is preferred that R is aryl, preferably an electron-rich aryl. An electron-rich aryl is meant to be an aryl with one or more electron donating substituent like $NH_2$, etc.

Preferably, $R^3$ is H, alkyl, alkaryl, or unsubstituted or substituted aryl. Preferably, $R^4$ is H, alkyl, alkaryl, or unsubstituted or substituted aryl. Preferably, $R^5$ is H, alkyl, alkaryl, or unsubstituted or substituted aryl.

Particularly, heteroaryl stands for pyridine, pyrimidine, triazine, furanyl, thiophenyl, etc.

Particularly, alkaryl stands for $-(CH_2)_mC_6H_5$, with m being 1, 2, 3 or 4.

In preferred embodiments, the compound according to Formula (I) is selected from the group consisting of:

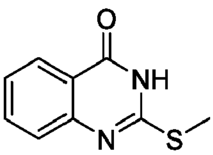,

(Compound BER384 (QSP265, GOL102))

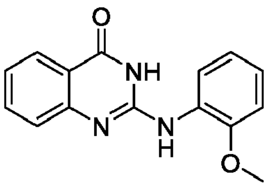

(Compound C123 (BER287))

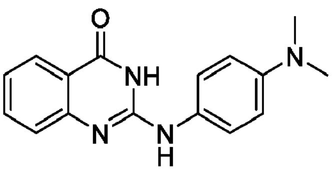,

(Compound C127 (BER263, BER294)

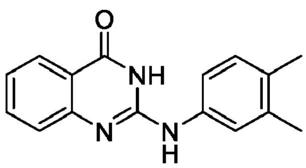,

(Compound C047 (QSP207))

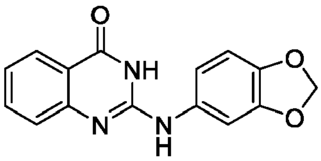

,

(Compound C128 (BER275, BER299)

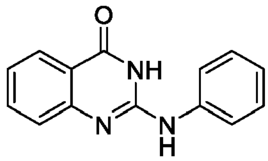

,

(Compound C126 (BER238))

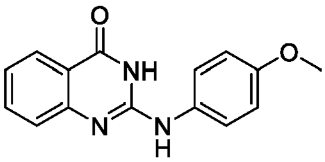

,

(Compound C121 (BER248, QSP206)

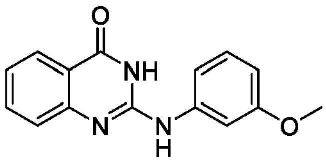

,

(Compound C122 (BER278, QSP221))

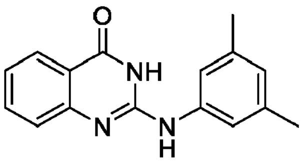

,

(Compound C060 (QSP220))

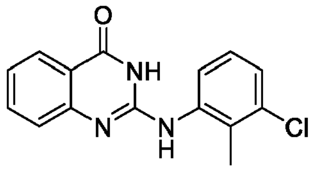

,

(Compound C130 (BER306))

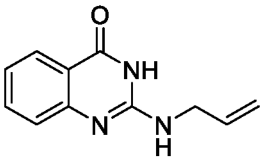

,

(Compound C043 (QSP203))

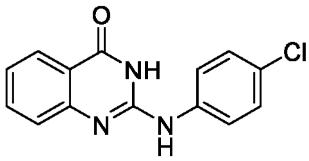

,

(Compound C124 (BER276, BER304))

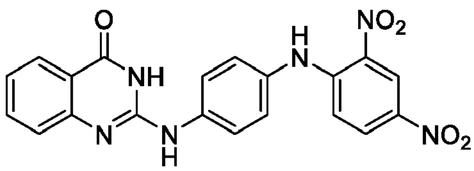

,

(Compound C172 (BER295))

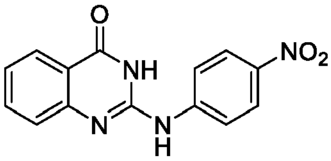

,

(Compound C129 (BER262, BER288))

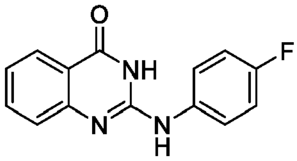

,

(Compound C125 (BER261, BER305))

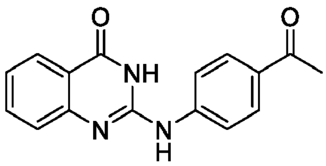
,

(Compound C178 (BER338))

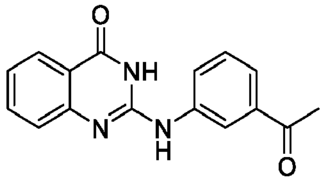
,

(Compound C179 (BER329, BER389))

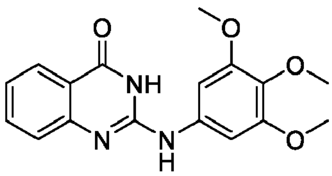
,

(Compound C173 (BER300))

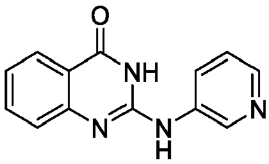
,

(Compound GOL192)

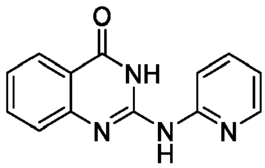
,

(Compound GOL193)

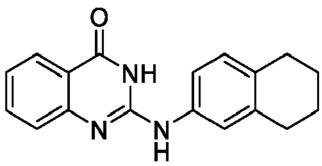
,

(Compound GOL194)

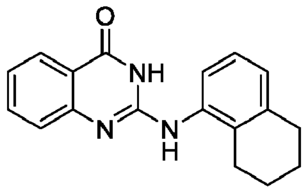
,

(Compound GOL195)

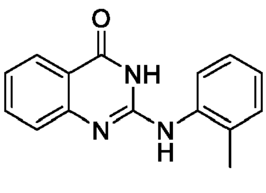
,

(Compound GOL298)

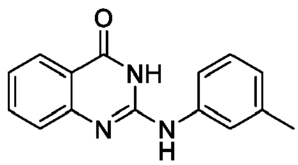
,

(Compound GOL299)

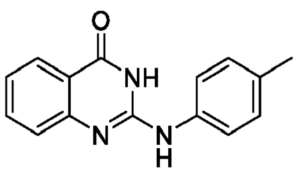
,

(Compound GOL300)

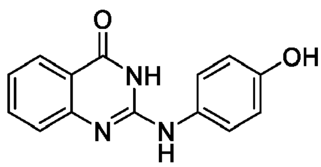
,

(Compound GOL321)

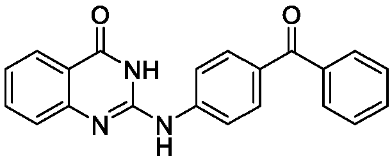,

(Compound GOL322)

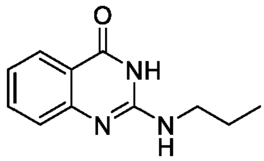,

(Compound C045 (QSP205))

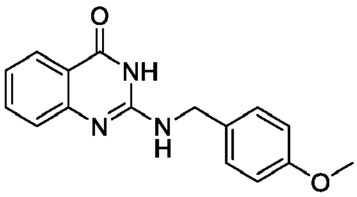,

(Compound GOL318)

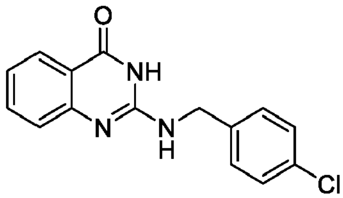,

(Compound GOL319)

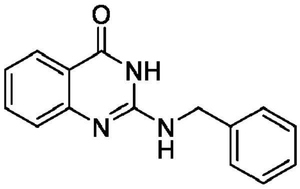,

(Compound GOL323)

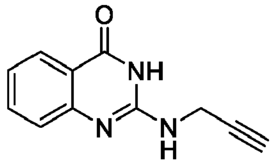,

(Compound GOL332)

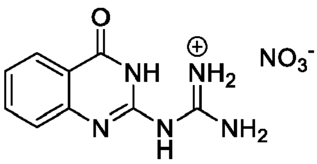,

(Compound BER255)

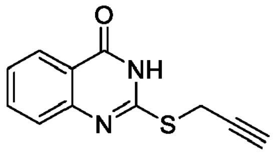,

(Compound C005 (GOL331, QSP165))

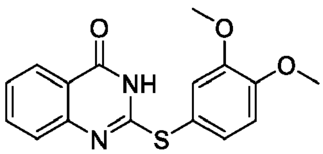,

(Compound GOL187)

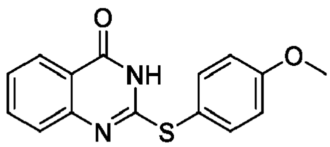,

(Compound GOL188)

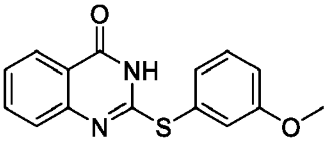,

(Compound GOL189)

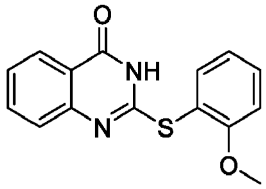,

(Compound GOL190)

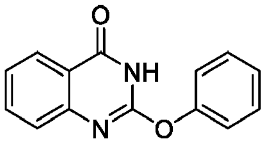,

(Compound GOL407)

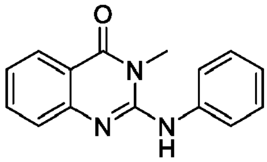

(Compound C176 (BER341))

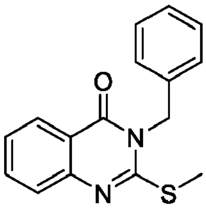

(Compound BER356)

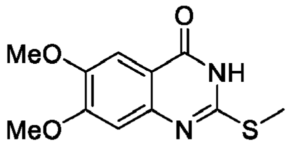,

(Compound BER365)

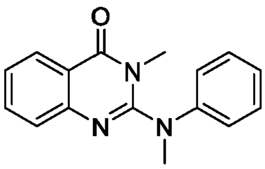,

(Compound C177 (BER330))

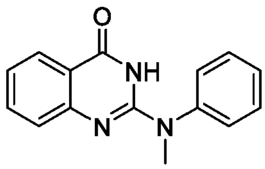,

(Compound C175 (BER308))

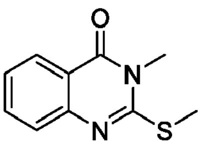,

(Compound BER319)

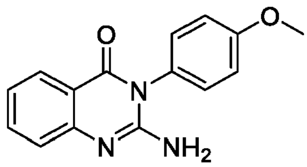,

(Compound BER220)

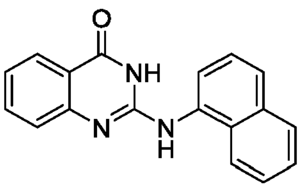,

(Compound BER321)

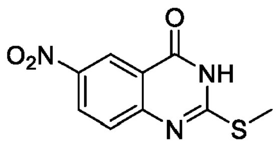,

(Compound BER407)

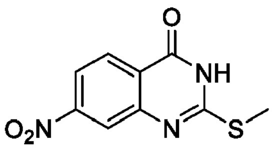,

(Compound BER408)

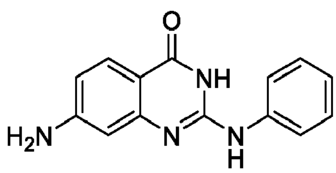,

(Compound C182 (BER419))

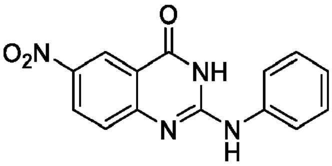
,

(Compound C181 (BER416))

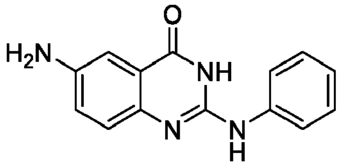
,

(Compound C183 (BER420))

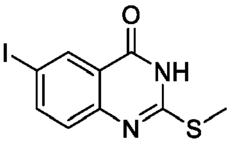
,

(Compound GOL374)

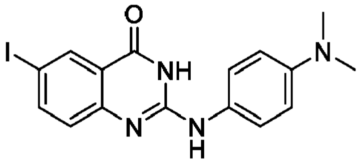
,

(Compound GOL375)

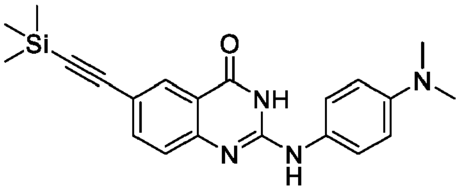
,

(Compound GOL385)

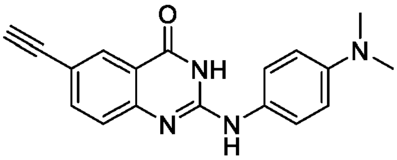
,

(Compound GOL389)

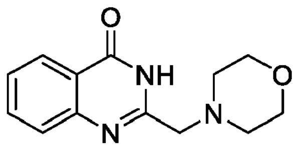

(Compound C023 (QSP183))

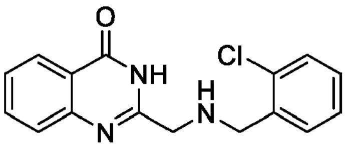
,

(Compound C028 (QSP188))

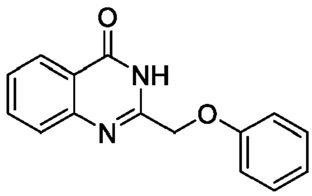
.

(Compound C024 (QSP184))

In further preferred embodiments, the compound according to Formula (I) is selected from the group consisting of the following compounds, as shown above:

Compound C123 (BER287),
Compound C127 (BER263, BER294),
Compound C047 (QSP207),
Compound C128 (BER257, BER299),
Compound GOL389,
Compound BER384 (QSP265, GOL102),
Compound C126 (BER238),
Compound C121 (BER248, QSP206),
Compound C122 (BER278, QSP221),
Compound C060 (QSP220),

Compound C130 (BER306), and
Compound C043 (QSP203).

More preferably, the compound according to Formula (I) is selected from the group consisting of the following compounds, as shown above:

Compound C123 (BER287),
Compound C127 (BER263, BER294),
Compound C047 (QSP207),
Compound C128 (BER257, BER299),
Compound GOL389,
Compound BER384 (QSP265, GOL102),
Compound C126 (BER238),
Compound C121 (BER248, QSP206), and
Compound C122 (BER278, QSP221).

Yet more preferably, the compound according to Formula (I) is selected from the group consisting of the following compounds, as shown above:

Compound C123 (BER287),
Compound C127 (BER263, BER294),
Compound C047 (QSP207),
Compound C128 (BER257, BER299),
Compound GOL389, and
Compound BER384 (QSP265, GOL102).

Yet more preferably, the compound according to Formula (I) is Compound C123 (BER287) or Compound BER384 (QSP265, GOL102), wherein Compound C123 (BER287) is most preferred.

In alternative embodiments, the compound according to Formula (I) is selected from the group consisting of: Compound GOL374, Compound GOL193, and Compound GOL190, wherein Compound GOL374 is particularly preferred.

The specific compounds of the present invention are labelled herein by internal designations, as indicated above. However, the respective IUPAC names can be easily determined from the respective structures by the person skilled in the art. The specific compounds of the present invention and their activity values in the different bioassays described in the Examples of the present application, are shown in Table 1.

Table 1: The specific compounds of the present invention and their activity values in the different bioassays described herein

| **Compound** | **Structure** | **Activity values in different bioassays** | |
|---|---|---|---|
| | | ***L. minor*** **(at 10 μM)** | **AtPARP inhibition in % at** |
| | | $\overline{d}_{tot,3} \pm CI_{d_{tot,3}}0,95$<br>$\overline{d}_{tot,4} \pm CI_{d_{tot4}}0,95$ | **1 μM**<br>**10 μM** |
| **BER384** | 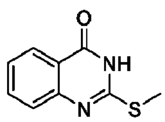 | n.d. | 23 ± 9<br>72 ± 13 |
| **BER287**<br>**C123** | 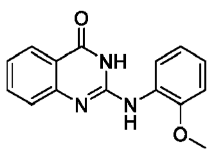 | 3.4 ± 1.0 | n.d. |

(continued)

| Compound | Structure | Activity values in different bioassays | |
|---|---|---|---|
| | | ***L. minor*** **(at 10 μM)** | **AtPARP inhibition in % at** |
| | | $\overline{d}_{tot,3} \pm CI_{d_{tot,3}}0{,}95$<br>$\overline{d}_{tot,4} \pm CI_{d_{tot4}}0{,}95$ | **1 μM**<br>**10 μM** |
| **BER263**<br>**C127** | 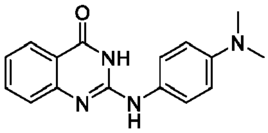 | 2.9 ± 0.9 | n.d. |
| **QSP207**<br>**C047** | 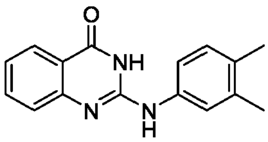 | 2.8 ± 1.7 | $IC_{50}$ 76 ± 1 μM |
| **BER275**<br>**C128** | 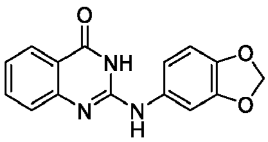 | 2.7 ± 0.9 | 9 ± 6<br>21 ± 6 |
| **BER238**<br>**C126** | 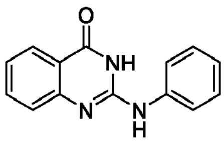 | 2.2 ± 0.8 | 2.0<br>12.6 ± 6 |
| **BER248**<br>**C121** | 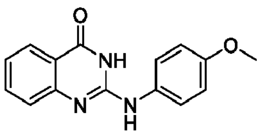 | 2.2 ± 0.8<br>2.3 ± 0.7 | -3.6 ± 13.5<br>5 ± 5<br>$IC_{50}$ 1,8 ± 0,3 μM |
| **BER278**<br>**C122** | 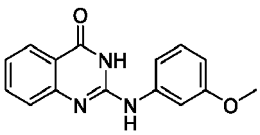 | 2.0 ± 0.8<br>2.6 ± 0.8 | 3.2 ± 3.5<br>15 ± 11<br>$IC_{50}$ 0.48 ± 0.16 μM |
| **QSP220**<br>**C060** | 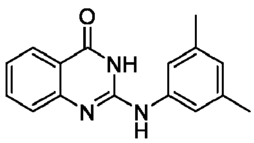 | 1.8 ± 1.4 | $IC_{50}$ >100 μM |
| **BER306**<br>**C130** | 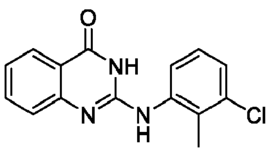 | 1.5 ± 0.8 | 4.2 ± 5.8<br>-9.5 ± 7.5 |
| **QSP203**<br>**C043** | 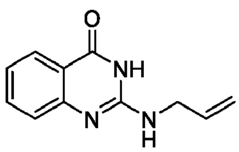 | 1.49 ± 1.3 | $IC_{50}$ 1.76 ± 1.5 μM |

(continued)

| Compound | Structure | Activity values in different bioassays | |
|---|---|---|---|
| | | *L. minor* (at 10 μM) | AtPARP inhibition in % at |
| | | $\overline{d}_{tot,3} \pm CI_{d_{tot,3}}0{,}95$<br>$\overline{d}_{tot,4} \pm CI_{d_{tot4}}0{,}95$ | 1 μM<br>10 μM |
| **BER276 C124** | 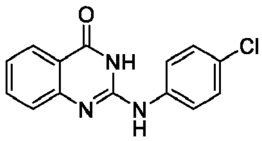 | 1.5 ± 0.7 | 22 ± 11.8<br>8.9 ± 15.4 |
| **BER295 C172** | 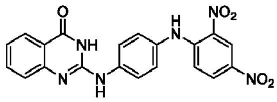 | 1.3 ± 0.7 | 7.5 ± 14<br>18.8 ± 4.3 |
| **BER262 C129** | 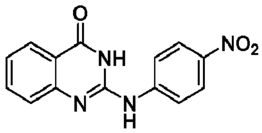 | 1.18 ± 0.7 | 22 ± 9<br>31.7 ± 7.8 |
| **BER261 C125** | 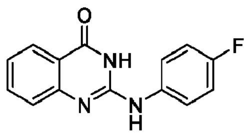 | 1.1 ± 0.7 | 8.3 ± 6.3<br>10.2 ± 8.6 |
| **BER338 C178** | 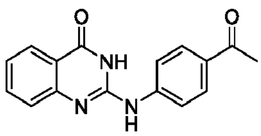 | 1.04 ± 0.6 (at 50 mM) | 6.1 ± 2.9<br>21.5 ± 11.1 |
| **BER329 C179** | 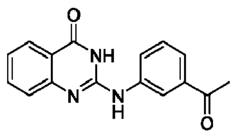 | 0.98 ± 0.71 (at 50 mM) | 20.9 ± 77<br>42 ± 22 |
| **BER300 C173** | 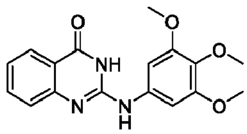 | approx. 0.50 (at 10 mM) at 100 μM 2.7 | 26 ± 10.6<br>50 ± 15 |
| **GOL192** | 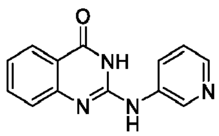 | -0.17 ± 0.07 | 9.4 ± 4<br>24 ± 25 |
| **GOL193** | 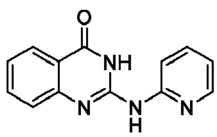 | -2.95 ± 0.04 | 14 ± 10.5<br>44 ± 42 |
| **GOL194** | 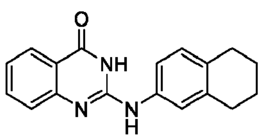 | -0.40 ± 0.02 | 2.3 ± 4.6<br>9.2 ± 9.6 |

(continued)

| Compound | Structure | Activity values in different bioassays | |
|---|---|---|---|
| | | *L. minor* (at 10 μM) | AtPARP inhibition in % at |
| | | $\overline{d}_{tot,3} \pm CI_{d_{tot,3}}0,95$<br>$\overline{d}_{tot,4} \pm CI_{d_{tot4}}0,95$ | 1 μM<br>10 μM |
| **GOL195** | 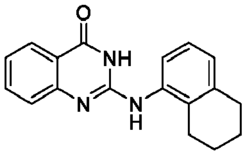 | -1.28 ± 0.11 | 3.54 ± 8.84<br>2.85 ± 5.88 |
| **GOL298** | 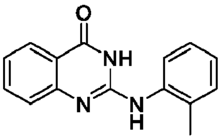 | 1.3 ± 0.07 | 13.7 ± 3.7<br>11.2 ± 2.7 |
| **GOL299** | 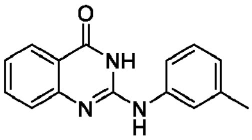 | n.d. | 15 ±3.9<br>12.7 ± 4.4 |
| **GOL300** | 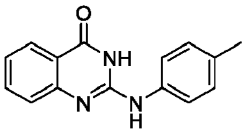 | -1.1 ± 0.05 | 0.5 ± 1.8<br>2 ± 2.9 |
| **GOL321** | 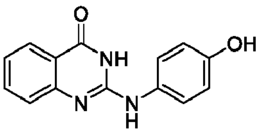 | 0.45 ± 0.04 | 38 ± 8<br>31.8 ± 6.6 |
| **GOL322** | 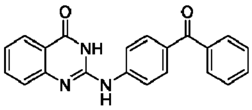 | n.d. | 7.8 ± 6.0<br>7.2 ± 1.7 |
| **QSP205 C045** | 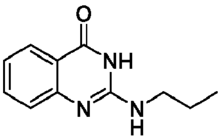 | 0.5 ± 1.17 | $IC_{50}$ 58.1 ± 1.1 μM |
| **GOL318** | 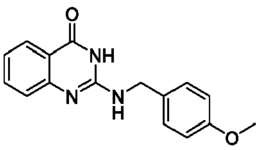 | 0.56 ± 0.06 | 23.8 ± 15.5<br>24.3 ± 22.8 |
| **GOL319** | 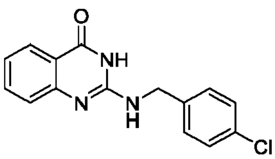 | -0.46 ± 0.06 | 23.7 ± 6.1<br>19.3 ± 4.9 |

(continued)

| Compound | Structure | Activity values in different bioassays | |
|---|---|---|---|
| | | *L. minor* (at 10 μM) | AtPARP inhibition in % at |
| | | $\overline{d}_{tot,3} \pm CI_{d_{tot,3}}0,95$<br>$\overline{d}_{tot,4} \pm CI_{d_{tot4}}0,95$ | 1 μM<br>10 μM |
| GOL323 | 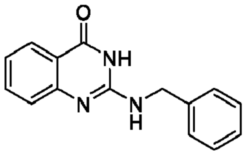 | 0.15 ± 0.03 | 16.6 ± 8.8<br>9.3 ± 4.4 |
| GOL332 | 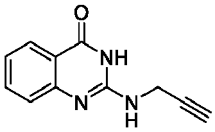 | 1.0 ± 0.02 | n.d. |
| BER255 | 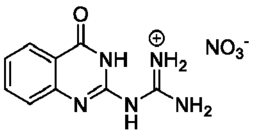 | n.d. | 12.4 ± 9.1<br>38.9 ± 10.2 |
| GOL331<br>C005 | 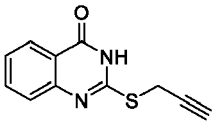 | 0.79 ± 1.20 | $IC_{50}$ 15.5 ± 1.3 μM |
| GOL187 | 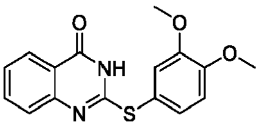 | 1.06 ± 0.03 | 3.02 ± 9.10<br>5.34 ± 12.72 |
| GOL188 | 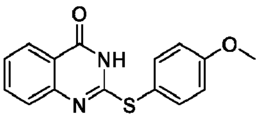 | 0.15 ± 0.03 | 3.3 ± 3.6<br>6.5 ± 6.4 |
| GOL189 | 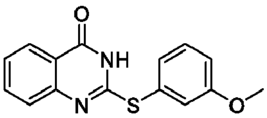 | -0.64 ± 0.02 | 5.3 ± 3.2<br>5 ± 4.3 |
| GOL190 | 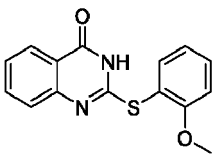 | -1.24 ± 0.02<br>-5.7 (50 μM) | 6.4 ± 4.2<br>11.4 ± 6.5 |
| GOL407 | 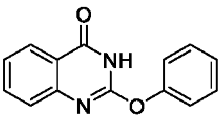 | 0.17 ± 0.05 | n.d. |
| BER341<br>C176 | 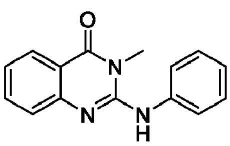 | 1.56 ± 1.29 (100 μM) | 5.6 ± 5.9<br>5.9 ± 5.4 |

(continued)

| Compound | Structure | Activity values in different bioassays | |
|---|---|---|---|
| | | *L. minor* (at 10 μM) | AtPARP inhibition in % at |
| | | $\bar{d}_{tot,3} \pm CI_{d_{tot,3}}0{,}95$<br>$\bar{d}_{tot,4} \pm CI_{d_{tot4}}0{,}95$ | 1 μM<br>10 μM |
| **BER356** | 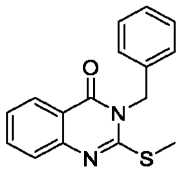 | n.d. | 10.0 ± 7.5<br>11.4 ± 13.4 |
| **BER365** | 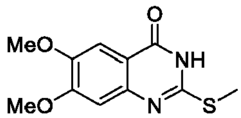 | n.d. | 7.2 ± 5.8<br>9.5 ± 4.7 |
| **BER330 C177** | 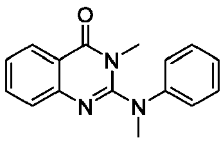 | 1.3 ± 1.2 (at 100 μM) | 4.5 ± 2.3<br>3.3 ± 7.2 |
| **BER308 C175** | 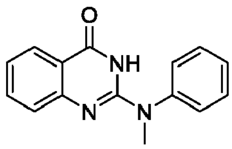 | 0.40 ± 1.14 (at 100 μM) | 2.4 ± 4.7<br>8.3 ± 3.1 |
| **BER319** | 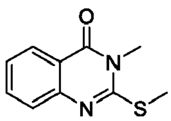 | n.d. | -4.7 ± 7<br>1.2 ± 5.9 |
| **BER220** | 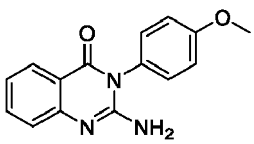 | n.d. | 6.3 ± 3.8<br>10.3 ± 4.7 |
| **BER321** | 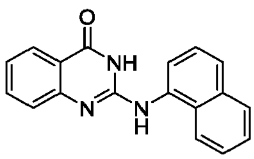 | n.d. | 14 ± 6.7<br>44.3 ± 14.4 |
| **BER407** | 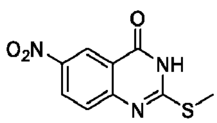 | n.d. | -2.5 ± 6<br>-1.1 ± 7.3 |
| **BER408** | 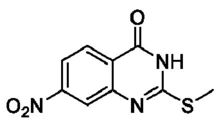 | n.d. | 3.3 ± 7.0<br>0.1 ± 6.0 |
| **BER419 C182** | 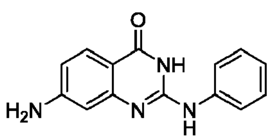 | 2.5 ± 1.07 (at 50 μM) | 7.8 ± 8.8<br>35 ± 7.8 |

(continued)

| Compound | Structure | Activity values in different bioassays | |
|---|---|---|---|
| | | ***L. minor*** **(at 10 μM)** | **AtPARP inhibition in % at** |
| | | $\bar{d}_{tot,3} \pm CI_{d_{tot,3}}0{,}95$<br>$\bar{d}_{tot,4} \pm CI_{d_{tot4}}0{,}95$ | **1 μM**<br>**10 μM** |
| **BER416**<br>**C181** | 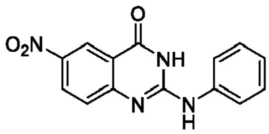 | 0 (50 μM) | -1.8 ± 7.2<br>-1.29 ± 7.06 |
| **BER420**<br>**C183** | 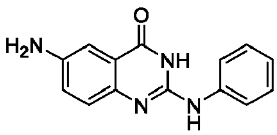 | 1.72 ± 0.94 (at 50 μM) | 6.4 ± 6.9<br>18.4 ± 5.3 |
| **GOL374** | 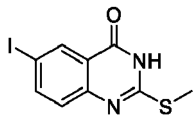 | -4.4 ± 0.3 | n.d. |
| **GOL375** | 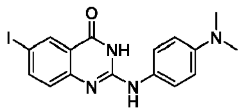 | 1.14 ± 0.04 | n.d. |
| **GOL385** | 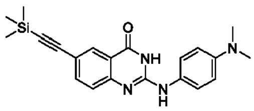 | 0.2 ± 0.03 | n.d. |
| **GOL389** | 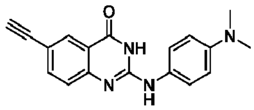 | 2.9 ± 0.05 | n.d. |
| **QSP183**<br>**C023** | 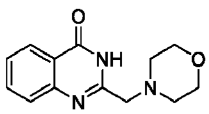 | 1.2 ± 1.26 | 20 ± 1.1 |
| **QSP188**<br>**C028** | 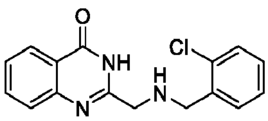 | 1.08 ± 1.2 at the recovery phase: 1.4 ± 1.3 | 4.6 ± 1.2 |
| **QSP184**<br>**C024** | 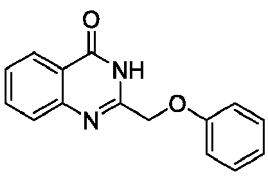 | 1.05 ± 1.24 at the recovery phase: 1.62 ± 1.36 | 31 ± 0.1 |
| n.d.: no data | | | |

The term "phytoeffector" as used herein relates to compounds acting on plants by inducing adaptive modifications of the plants. Particular effects such phytoeffectors can have on plants include:

(i) increasing plant growth,

(ii) increasing plant health,

(iii) increasing plant biomass,

(iv) increasing plant yield,

(v) decreasing plant growth,

(vi) inhibiting plant growth,

(vii) abolishing pant growth,

(viii) killing the plant, and

(vii) inhibiting plant PARP enzymes.

In specific embodiments, respective uses according to the present invention include the plant undergoing abiotic stress, in particular drought stress.

In a second aspect, the present invention relates to a compound, selected from the group consisting of

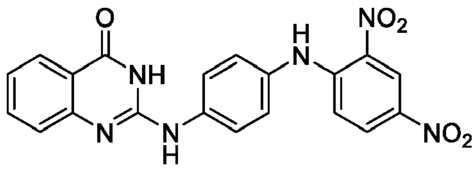
,

(Compound C172 (BER295))

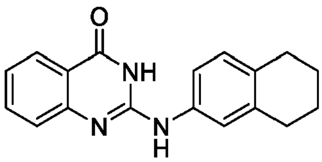
,

(Compound GOL194)

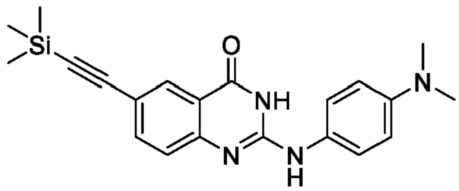
,

(Compound GOL385)

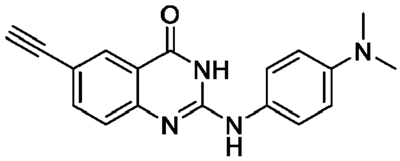
.

(Compound GOL389)

As above, the specific compounds of the present invention are labelled herein by internal designations, as indicated above. However, the respective IUPAC names can be easily determined from the respective structures by the person skilled in the art.

In a third aspect, the present invention relates to the use of a compound according to Formula (II) as a phytoeffector:

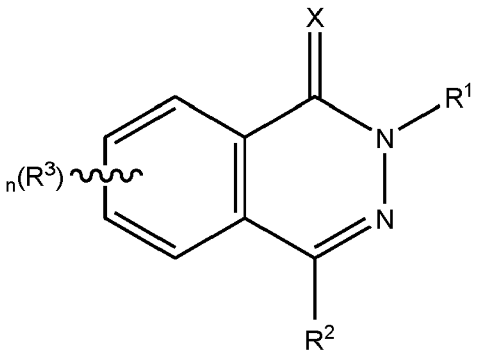

(II)

wherein

X is O, N-$NR^4_2$, N-$OR^4$, or $NR^4$, particularly O,

$R^1$ is H, alkyl, or unsubstituted or substituted alkaryl or aryl, particularly H,

$R^2$ is unsubstituted or substituted aryl, unsubstituted or substituted alkaryl, unsubstituted or substituted amino, or $CH_2$NH-aryl which can be unsubstituted or substituted on the aryl moiety;

$R^3$ is H, halogen, nitro, unsubstituted or substituted amino, alkyl, alkoxy, unsubstituted or substituted alkynyl or unsubstituted or substituted alkaryl or aryl; or $R^3$ stands for one or more annulated aromatic rings to form a naphthalene, anthracene, or phenanthrene moiety,

$R^4$ is H, alkyl, or unsubstituted or substituted aryl or alkaryl, and

n is 0, 1, 2, 3, or 4,

where the aforementioned substituent(s) can be selected from the group consisting of halogen, alkyl, alkoxy, aryloxy, trialkylsilyl, $SO_2N(CH_3)_2$, nitro, unsubstituted or substituted amino including aminoheterocycles such as e.g. morpholino.

In preferred embodiments, the compound according to Formula (II) is selected from the group consisting of:

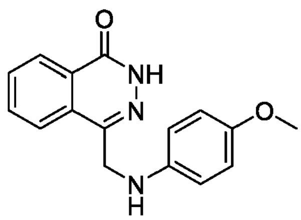

,

(Compound QSP192 (C032))

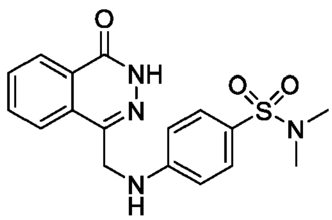

,

(Compound QSP256 (C096))

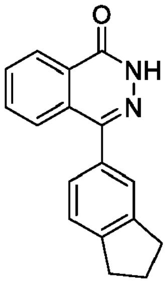

,

(Compound QSP252 (C092))

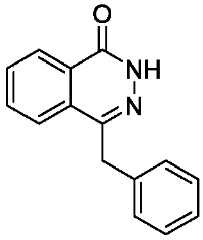

,

(Compound QSP255 (C095))

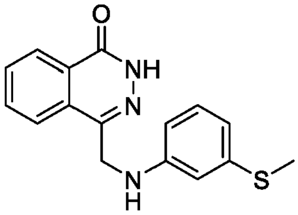

,

(Compound QSP210 (C050))

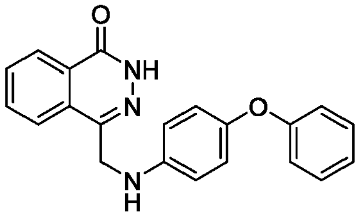

,

(Compound QSP235 (C075))

The specific compounds of the present invention are labelled herein by internal designations, as indicated above. However, the respective IUPAC names can be easily determined from the respective structures by the person skilled in the art.

The term "phytoeffector" as used herein relates to compounds acting on plants by inducing adaptive modifications of the plants. Particular effects such phytoeffectors can have on plants include:

(i) increasing plant growth,
(ii) increasing plant health,
(iii) increasing plant biomass,
(iv) increasing plant yield,
(v) decreasing plant growth,
(vi) inhibiting plant growth,
(vii) abolishing pant growth,
(viii) killing the plant, and
(vii) inhibiting plant PARP enzymes.

In specific embodiments, respective uses according to the present invention include the plant undergoing abiotic stress, in particular drought stress.

As used herein, the term "about" represents a modifier of ± 10% of the specified value, preferably ± 7.5%, ± 5%, ± 3%, ± 2%, or ± 1% of the specified value. Thus, by way of example, the term "about 10" includes the ranges 9 to 11, 9.25 to 10.75, 9.5 to 10.5, 9.7 to 10.3, 9.8 to 10.2, and 9.9 to 10.1.

The present invention sought to deepen the knowledge about drought stress tolerance-inducing effects and the underlying mode(s) of action(s) believed to be present in selected classes of synthetic chemical compounds different from the known ones described so far. Previously conducted research based on the hypothesis of PARP enzymes being involved in plant stress response yielded a hypothetic mode of action and a selection of promising synthetic compounds found through *in silico* screening. In order to provide experimental proof, an *in vivo* assay system was developed and first evidence of the desired activity being inherent to different compounds was documented.

The first aim of the present invention was to confirm the described effects and to expand the phenotypical screening onto additional structural classes, including diverse heterocyclic compounds. Therefore, the established assay system was to be improved in efficiency, allowing for shorter screening time, thus enabling faster and broader screening of compound libraries. Potentially active compound classes have then been investigated in depth by syntheses of various new derivatives and subsequent assessment of their activity. Thereby, special emphasis was put onto the development of a robust, cost-effective and upscalable synthetic access to the desired compounds in order to facilitate an industrial-scale application.

The present invention aims at the identification of drought stress tolerance-inducing effects in small molecules.

Aiming to confirm and extend previously gained knowledge, the main objective of the invention lay in the investigation of a possible connection between plant PARP enzyme inhibition and an increase in drought stress tolerance induction.

Starting from the original setup of the *Lemna minor* bioassay (Thorsten Geißler, Ludger A. Wessjohann, "A Whole-Plant Microtiter Plate Assay for Drought Stress Tolerance-Inducing Effect", Journal of Plant Growth Regulation 30.4 (2011), pp.504-511), several improvements were implemented, targeting at a reduction in required time and manual labour, as well as a higher robustness in statistical evaluation and reliability of obtained results. Compound were additionally assayed with *Arabidopsis thaliana* and *Lollium perenne.*

The figures show:

Figure 1:
Synthetic route to quinazolinones with core-amino-substitution (for consistency, position numbering of nitroanthranilic acids **37** follows IUPAC rules for the final product here; correct numbering is applied in the experimental section) a: MeOH, $H_2SO_4$, reflux (quantitative); b: KSCN, PhCOCl, acetone; c: 3 % NaOMe in MeOH (**39b:** 98 %; **39c:** 62 % recryst.); d: MeI, NaH, DMF (**40b**: 38 % recryst.; **40c:** 94 % recryst.); e: PhNH2 , AcOH (**41b**: 69 % recryst.; **41c:** 79 % recryst.); f: Fe, AcOH, EtOH (**42b**: 16 % chrom.; **42c:** 30 % chrom.)

Figure 2:
Results of mean effect sizes of three-fold measurement. Mean total relative growth effect size $\overline{d} \pm CI_{\overline{d}tot,3,0,95}$ for 10 compounds tested at 10 μM concentration ranked according to effect size; highlighted vertical lines indicate effect size levels for $n$ = 3 replicates.

Figure 3:
Structure and concentration-activity profile of compound **C126.**

Figure 4:
Structure and concentration-activity profile of compound **C184.**

Figure 5:
Structure and concentration-activity profile of compounds **C121**, **C122** and **C123.**

Figure 6:
Structure and concentration-activity profile of compounds **C128**, **C173** and **C127.**

Figure 7:
Ranking of synthesized electron-rich quinazolinone derivatives according to effects exhibited in the *in vivo* assay at 10 μM and 25 μM concentration.

Figure 8:
Structure and concentration-activity profile of compounds **C124**, **C125**, **C129.**

Figure 9:
Structure and concentration-activity profile of compounds **C178** and **C179.**

Figure 10:
Structure and concentration-activity profile of compounds **C130**, **C174** and **C172.**

Figure 11:
Structure and concentration-activity profile of compounds **C175**, **C176** and **177.**

Figure 12:
Structure and concentration-activity profile of compounds **C180** and **C182.**

Figure 13:
Structure and concentration-activity profile of compounds **C181** and **C183.**

Figure 14:
Assessment of growth enhancement under stress and non-stress conditions demonstrated by single application of 100 μM **C126** at $t_0$; shown is the logarithmic frond area $\ln A \pm CI_{0.95}$ for groups of $n$ = 6 plants, dashed line: negative

control (DMSO), solid line: test group left: Non-stress conditions (without PEG); middle: Stress conditions (full strength PEG); right: Stress application for 2 d followed by recovery for 3 d.

The present invention will be further illustrated by the following examples without being limited thereto.

## Examples

Material and methods:

*Instrumentation*

Recording of *Lemna minor* assay images was carried out on a LemnaTec Scanalyser system using the built-in software. Images of size 1280 × 980 px were exported to the computer's hard drive. Batch analysis of image series was carried out using the software Imaged (version 1.49v) using an appropriate algorithm.

Analytical thin-layer chromatography was conducted with silica coated aluminium plates (silica 60 *F*254) obtained from Merck, Germany. Compound detection was achieved either by UV irradiation (254 nm) or staining and subsequent heating (> 100 °C) using cerium(IV)- molybdatophosphoric acid.

One- and two-dimensional NMR spectra were recorded on Varian Mercury 300, 400 and 600 spectrometers. Chemical shifts are reported relative to tetramethylsilane (TMS) ($\delta$ = 0.00) for 1H spectra and DMSO ($\delta$ = 39.52) or chloroform ($\delta$ = 77.16) for 13C spectra. Signal multiplicities are reported using the following abbreviations: s (singlet), d (duplet), t (triplet), q (quartet), m (multiplet).

Standard mass spectra were recorded on an API-150 device (Applied Biosystems) in positive and negative electrospray mode. High resolution mass spectra in both positive and negative mode were recorded either on a Bruker Apex III Fourier transform ion cyclotron resonance (FT-ICR) mass spectrometer (Bruker Daltonics, Billerica, USA) equipped with an external APOLLO electrospray ion source (Agilent Technologies, Santa Clara, USA), or an Orbitrap Elite mass spectrometer (Thermofisher Scientific, Bremen, Germany) equipped with an HESI electrospray ion source in positive or negative electrospray mode.

HPLC traces were recorded on a VWR Hitachi LaChrom Elite instrument (L-2 series) using a YMC C-18 column (150 × 4.6 mm, particle size: 5 μm, pore size: 120 Å). The eluents used were MeCN + 0.1 % TFA (A) and water + 0.1 % TFA (B) at a flow rate of 0.8 ml/min with the following gradient: 5 % A (4 min) / 5 % A → 100 % A (15 min) / 100 % A (5 min). Detection of the analytes was performed in the UV range at 254 nm. Signals present in the solvent blank were excluded upon determination of analyte purity.

*Microtiter plate assay with* Lemna minor - *Culture medium and maintenance*

Axenic cultures of *Lemna minor* were originally provided by the Canadian Phycological Culture Centre (strain number CPCC 490). Cultures were maintained under sterile conditions in 500 ml Erlenmeyer flasks, equipped with cellulose plugs in a Sanyo MLR-351H Environmental Test Chamber at constant illumination (~ 120 $\mu mol \cdot m^{-2} \cdot s^{-1}$, 27 °C). Every seven days, 10- 15 plants were transferred to a new flask containing 200 ml of sterile STEINBERG medium. For the preparation of one litre of STEINBERG medium, 20 ml each of stock solutions 1 - 3 were mixed with 1 ml each of stock solutions 4 and 5. The mixture was diluted to 950 ml with distilled water, the pH was adjusted to 5.5 with 1 M hydrochloric acid, the solution was filled up to 1000 ml with distilled water and sterilized.

*Microtiter plate assay with* Lemna minor - *Stress medium*

For the preparation of one litre of STEINBERG stress medium, the stock solutions were mixed as described above and diluted to 500 ml. Under stirring, 150 g PEG6000 were dissolved and the solution was further diluted to 950 ml with distilled water. After adjusting the pH to 5.5, the solution was filled up to 1000 ml and sterilized by suction filtration through a bottle top filter (PES, 0.2 μm pore size).

*Microtiter plate assay with* Lemna minor - *Preparation and maintenance of assay plates*

A 24-well microtiter plate was divided horizontally and vertically into four groups of six wells each. Each well was equipped with 2 ml of stress medium and 2 μl of test substance (DMSO for control or compound stock in DMSO), resulting in a final DMSO concentration of 0.1 %. After all wells were filled, 20 ml of stress medium was spread within the cavity between the wells to reduce water evaporation from the medium within the wells. For each plate, three-fronded plants of comparable size were taken from a single culture flask using an inoculation loop and were distributed randomly across the wells. The plate was closed and the lid was fixed with tape. Subsequently, the first image was taken. After incubation for 48

h the plate was opened, the wells were carefully drained using a pipette (Eppendorf, Germany) and refilled with 2 ml of PEG-free STEINBERG medium after which the second image was taken. After further incubation for 24 h the third image was taken.

In a variation of the assay, 12-well plates were used, containing 4 µl of test substance (or DMSO) in 4 ml medium. Application of this setup is indicated in the text.

*Microtiter plate assay with* Lemna minor - *Imaging of assay plates, processing and statistical analysis*

The following processing steps were applied to all assay images using the software ImageJ:

- enhance contrast
- split channels, select blue channel
- set threshold (method: "Intermodes")
- analyze particles (size: 900 - ∞, circularity: 0.10 - 1.00)

The results containing x and y coordinates as well as frond areas for each plant were saved into a text file.

*Synthetic procedures - 2-(Arylamino)-quinazolin-4(3H)-ones*

A solution of 2-(Methylthio)-quinazolin-4(3*H*)-one **(25, BER239)** (961 mg, 5.0 mmol) and aryl amine (7.5 mmol) in glacial acetic acid (7.5 ml) was heated to reflux under stirring, while checking conversion of starting materials by TLC. Where indicated, additional 2.5 mmol of aryl amine were added within the first 2 h of the reaction. If product started to precipitate during the course of the reaction, acetic acid was added dropwise to keep the reaction mixture from solidifying. After the indicated time, the heating was removed and water was added dropwise under stirring until a fine precipitate formed. The slurry was cooled to room temperature and additional water was added until a volume of 80 - 90 ml was reached. The mixture was cooled in the fridge for at least 3 h, the crude product was filtered off.

If the filtrate was free of product (judged by ESI-MS), the crude product was dried *in vacuo* and directly recrystallized from solvent mixtures. If product could still be found in the filtrate, it was extracted once with dichloromethane and ethyl acetate (125 ml each). The crude product and the organic phases were combined, the solvent was removed *in vacuo* and pure products were obtained by recrystallization.

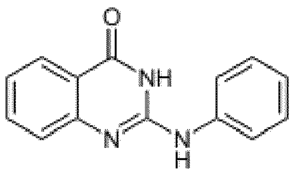

**2-(Phenylamino)-quinazolin-4(3H)-one (31a, BER291)**

**Yield** 79 % (white solid);
**1H-NMR** (400 MHz, DMSO-d6) $\delta$ 10.80 (s, 1 H), 8.64 (s, 1H), 7.97 (dd, *J* = 7.9, 1.0 Hz, 1 H), 7.75 (d, *J* = 7.9 Hz, 2 H), 7.69 - 7.62 (m, 1 H), 7.41 (d, *J* = 8.1 Hz, 1 H), 7.36 (t, *J* = 7.9 Hz, 2 H), 7.27 - 7.20 (m, 1 H), 7.05 (t, *J* = 7.3 Hz, 1 H); **13C-NMR** (100 MHz, DMSO-d6) $\delta$ 161.55, 149.98, 147.35, 138.94, 134.43, 128.81, 125.85, 125.32, 123.03, 122.45, 119.26, 118.36; **HR-MS** *m/z* 236.0829 (found), 236.0829 (calc.) [M-H] ; **Purity** (HPLC, 254 nm) 99.3%;

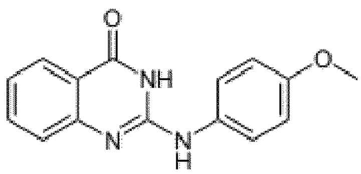

**2-((4-Methoxyphenyl)amino)-quinazolin-4(3H)-one (31b, BER292)**

**Yield** 94 % (off-white solid);
**1H-NMR** (400 MHz, DMSO-d6) $\delta$ 10.76 (s, 1 H), 8.46 (s, 1 H), 7.95 (dd, *J* = 8.0, 1.2 Hz, 1 H), 7.69 - 7.54 (m, 3 H), 7.34 (d, *J* = 8.1 Hz, 1 H), 7.20 (t, *J* = 7.4 Hz, 1 H), 6.98 - 6.89 (m, 2 H), 3.75 (s, 3 H); **13C-NMR** (100 MHz, DMSO-d6) $\delta$ 161.69, 155.04, 150.26, 147.75, 134.34, 131.81, 125.88, 125.11, 122.66, 121.41, 118.14, 114.01, 55.21; **HR-MS** *m/z* 266.0926 (found), 266.0935 (calc.) [M-H]-; **Purity** (HPLC, 254 nm) 98.9%;

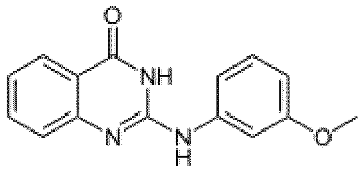

**2-((3-Methoxyphenyl)amino)-quinazolin-4(3H)-one (31c, BER293)**

**Yield** 88 % (off-white solid);
**1H-NMR** (400 MHz, DMSO-d6) $\delta$ 10.77 (s, 1 H), 8.66 (s, 1 H), 7.97 (dd, *J* = 7.8, 1.0 Hz, 1 H), 7.71 - 7.63 (m, 1 H), 7.60 (s, 1 H), 7.42 (d, *J* = 8.1 Hz, 1 H), 7.29 - 7.21 (m, 2 H), 7.15 (d, *J* = 8.1 Hz, 1 H), 6.63 (dd, *J* = 8.1, 2.0 Hz, 1 H), 3.79 (s, 3 H); **13C-NMR** (100 MHz, DMSO-d6) $\delta$ 161.50, 159.64, 149.87, 147.25, 140.12, 134.46, 129.54, 125.84, 125.38, 123.12, 118.37, 111.45, 107.83, 105.07, 54.97; **HR-MS** *m/z* 266.0938 (found), 266.0935 (calc.) [M-H] ; **Purity** (HPLC, 254 nm) 99.2 %;

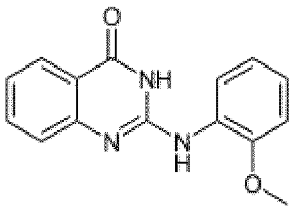

**2-((2-Methoxyphenyl)amino)-quinazolin-4(3H)-one (31d, BER287)**

**Yield** 79 % (white solid);
**1H-NMR** (400 MHz, DMSO-d6) $\delta$ 11.46 (s, 1 H), 8.69 (d, *J* = 8.6 Hz, 1 H), 8.35 (s, 1 H), 7.97 (dd, *J* = 7.8, 0.9 Hz, 1 H), 7.70 - 7.62 (m, 1 H), 7.43 (d, J = 8.2 Hz, 1 H), 7.29 - 7.19 (m, 1 H), 7.10 - 6.95 (m, 3 H), 3.91 (s, 3 H); **13C-NMR** (100 MHz, DMSO-*d*6) $\delta$ 161.59, 149.97, 148.08, 147.42, 134.38, 127.99, 125.82, 125.38, 123.04, 122.46, 120.51, 119.39, 118.40, 110.74, 55.92; **HR-MS** *m/z* 266.0936 (found), 266.0935 (calc.) [M-H] ; **Purity** (HPLC, 254 nm) 98.9%;

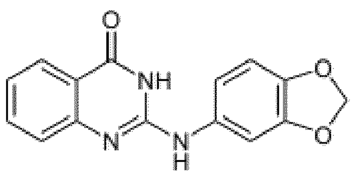

**2-((3,4-Methylenedioxyphenyl)amino)-quinazolin-4(3H)-one (31e, BER299)**

**Yield** 65 % (off-white solid);
**1H-NMR** (400 MHz, DMSO-*d6*) $\delta$ 10.75 (s, 1 H), 8.52 (s, 1 H), 7.95 (dd, *J* = 7.9, 1.0 Hz, 1 H), 7.68 - 7.59 (m, 1 H), 7.54 (d, *J* = 1.9 Hz, 1 H), 7.37 (d, *J* = 8.0 Hz, 1 H), 7.25 - 7.17 (m, 1 H), 6.98 (dd, *J* = 8.4, 2.0 Hz, 1 H), 6.89 (d, *J* = 8.3 Hz, 1 H), 6.02 (s, 2 H); **13C-NMR** (100 MHz, DMSO-d6) $\delta$ 161.58, 150.07, 147.60, 147.18, 142.65, 134.41, 133.17, 125.84, 125.18, 122.81, 118.22, 112.49, 108.09, 102.22, 101.00; **HR-MS** *m/z* 280.0730 (found), 280.0728 (calc.) [M-H] ; **Purity**

(HPLC, 254 nm) 98.9 %;

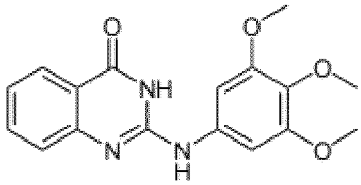

**2-((3,4,5-Trimethoxyphenyl)amino)-quinazolin-4(3H)-one (31f, BER300) Yield** 43 % (white solid);

**1H-NMR** (400 MHz, DMSO-*d*6) δ 10.77 (s, 1 H), 8.61 (s, 1 H), 7.97 (dd, *J* = 7.9, 0.9 Hz, 1 H), 7.69 - 7.62 (m, 1 H), 7.41 (d, *J* = 8.0 Hz, 1 H), 7.26 - 7.20 (m, 1 H), 7.14 (s, 2 H), 3.81 (s, 6 H), 3.64 (s, 3 H); **13C-NMR** (100 MHz, DMSO-d6) δ 161.58, 152.77, 149.88, 147.36, 134.95, 134.44, 132.87, 125.83, 125.43, 123.01, 118.29, 97.23, 60.12, 55.73; **HR-MS** *m/z* 326.1143 (found), 326.1146 (calc.) [M-H] ; **Purity** (HPLC, 254 nm) 99.1 %;

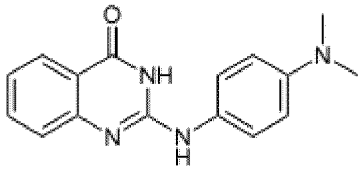

**2-((4-(Dimethylamino)phenyl)amino)-quinazolin-4(3*H*)-one (31g, BER294) Yield** 81 % (blue-grey solid);

**1H-NMR** (400 MHz, DMSO-d6) δ 10.69 (s, 1 H), 8.28 (s, 1 H), 7.93 (d, *J* = 7.2 Hz, 1 H), 7.66 - 7.55 (m, 1 H), 7.49 (d, *J* = 8.9 Hz, 2 H), 7.30 (d, *J* = 8.1 Hz, 1 H), 7.17 (t, *J* = 7.4 Hz, 1 H), 6.75 (d, *J* = 9.0 Hz, 2 H), 2.87 (s, 6 H); **13C-NMR** (100 MHz, DMSO-d6) δ 161.67, 150.52, 147.94, 146.96, 134.31, 128.32, 125.84, 125.00, 122.33, 121.60, 118.01, 112.88, 40.55; **HR-MS** *m/z* 279.1253 (found), 279.1251 (calc.) [M-H] ; **Purity** (HPLC, 254 nm) 98.2%;

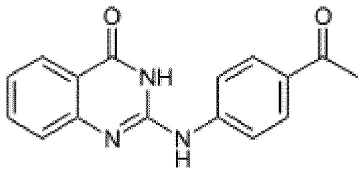

**2-((4-Acetylphenyl)amino)-quinazolin-4(3H)-one (31h, BER328)**

**Yield** 59 %, prepared in 15 mmol scale (yellowish solid);
**1H-NMR** (400 MHz, DMSO-d6) δ 10.93 (s, 1 H), 9.09 (s, 1 H), 8.03 - 7.85 (m, 5 H), 7.74 - 7.66 (m, 1 H), 7.48 (d, *J* = 8.1 Hz, 1 H), 7.33 - 7.26 (m, 1 H), 2.55 (s, 3 H); **13C-NMR** (100 MHz, DMSO-d6) δ 196.28, 161.49, 149.49, 146.86, 143.50, 134.55, 130.78, 129.56, 125.90, 125.55, 123.64, 118.68, 118.17, 26.39; **HR-MS** *m/z* 278.0933 (found), 278.0935 (calc.) [M-H] ; **Purity** (HPLC, 254 nm) 99.0 %;

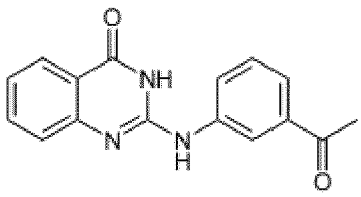

**2-((3-Acetylphenyl)amino)-quinazolin-4(3H)-one (31i, BER329)**

**Yield** 62 %, prepared in 15 mmol scale (brown solid);
**1H-NMR** (400 MHz, DMSO-*d*6) $\delta$ 10.90 (s, 1 H), 8.89 (s, 1 H), 8.38 (s, 1 H), 7.99 (d, *J* = 7.8 Hz, 2 H), 7.72 - 7.61 (m, 3 H), 7.51 (t, *J* = 7.9 Hz, 1 H), 7.42 (d, *J* = 8.1 Hz, 1 H), 7.29 - 7.23 (m, 1 H); **13C-NMR** (100 MHz, DMSO-d6) $\delta$ 197.66, 161.59, 149.72, 147.32, 139.41, 137.36, 134.51, 129.18, 125.89, 125.38, 123.77, 123.28, 122.27, 118.69, 118.49, 26.77; **HR-MS** *m*/*z* 280.1076 (found), 280.1081 (calc.) [M+H]+; **Purity** (HPLC, 254 nm) 98.2%;

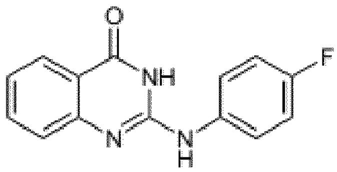

**2-((4-Fluorophenyl)amino)-quinazolin-4(3H)-one (31j, BER305)**

**Yield** 74 % (white solid);
**1H-NMR** (400 MHz, DMSO-d6) $\delta$ 10.84 (s, 1 H), 8.67 (s, 1 H), 7.97 (d, *J* = 7.7 Hz, 1 H), 7.75 (dd, *J* = 8.8, 4.9 Hz, 2 H), 7.68 - 7.61 (m, 1 H), 7.38 (d, *J* = 8.1 Hz, 1 H), 7.27 - 7.15 (m, 3 H); **13C-NMR** (100 MHz, DMSO-*d*6) $\delta$ 161.61, 157.69 (d, *J* = 239.1 Hz), 149.93, 147.50, 135.28, 134.43, 125.86, 125.23, 123.01, 121.25 (d, *J* = 7.8 Hz), 118.35, 115.33 (d, *J* = 22.3 Hz); **HR-MS** *m*/*z* 256.0885 (found), 256.0881 (calc.) [M+H]+; **Purity** (HPLC, 254 nm) 98.9%;

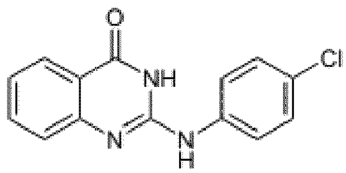

**2-((4-Chlorophenyl)amino)-quinazolin-4(3H)-one (31k, BER304)**

**Yield** 71 % (white solid);
**1H-NMR** (400 MHz, DMSO-d6) $\delta$ 10.86 (s, 1 H), 8.79 (s, 1 H), 7.98 (d, *J* = 7.6 Hz, 1 H), 7.78 (d, *J* = 8.6 Hz, 2 H), 7.70 - 7.63 (m, 1 H), 7.45 - 7.36 (m, 3 H), 7.25 (t, *J* = 7.3 Hz, 1 H); **13C-NMR** (100 MHz, DMSO-d6) $\delta$ 161.54, 149.74, 147.21, 137.97, 134.47, 128.62, 125.97, 125.87, 125.33, 123.24, 120.84, 118.45; **HR-MS** *m*/*z* 270.0442 (found), 270.0440 (calc.) [M-H] ; **Purity** (HPLC, 254 nm) 99.2 %;

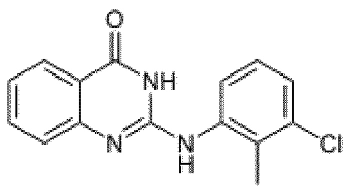

**2-((3-Chloro-2-methylphenyl)amino)-quinazolin-4(3H)-one (31l, BER306)**

**Yield** 73 % (off-white solid);
**1H-NMR** (400 MHz, DMSO-d6) $\delta$ 11.25 (s, 1 H), 8.15 (s, 1 H), 7.96 (d, *J* = 7.3 Hz, 2 H), 7.65 -7.57 (m, 1 H), 7.30 (d, *J* = 8.1 Hz, 1 H), 7.27 -7.18 (m, 3 H), 2.31 (s, 3 H); **13C-NMR** (100 MHz, DMSO-*d*6) $\delta$ 161.75, 149.98, 148.17, 138.41, 134.34, 133.62, 128.31, 127.00, 125.89, 125.11, 124.75, 122.92, 122.49, 118.28, 14.85; **HR-MS** *m*/*z* 284.0600 (found), 284.0596 (calc) [M-H] ; **Purity** (HPLC, 254 nm) 99.1 %;

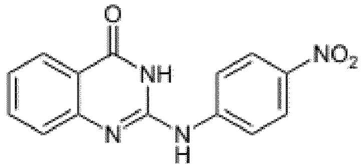

**2-((4-Nitrophenyl)amino)-quinazolin-4(3H)-one (31m, BER288)**

**Yield** 46 % (yellow solid);
**1H-NMR** (400 MHz, DMSO-d6) δ 11.05 (s, 1 H), 9.42 (s, 1 H), 8.28 - 8.21 (m, 2 H), 8.06 - 7.97 (m, 3 H), 7.76 - 7.69 (m, 1 H), 7.51 (d, *J* = 8.1 Hz, 1 H), 7.32 (t, *J* = 7.3 Hz, 1 H); **13C-NMR** (100 MHz, DMSO-*d*6) δ 161.46, 149.14, 146.58, 145.52, 141.24, 134.62, 125.93, 125.68, 125.04, 124.07, 118.89, 118.47; **HR-MS** *m*/*z* 281.0680 (found), 281.0680 (calc.) [M-H] ; **Purity** (HPLC, 254 nm) 100.0 %;

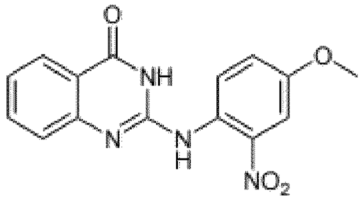

**2-((4-Methoxy-2-nitrophenyl)amino)-quinazolin-4(3H)-one (31n, BER315)**

**Yield** 56 % (orange needles);
**1H-NMR** (400 MHz, DMSO-*d*6) δ 11.87 (s, 1 H), 9.21 (s, 1 H), 8.29 (s, 1 H), 7.97 (d, *J* = 7.7 Hz, 1 H), 7.67 - 7.61 (m, 1 H), 7.59 (d, *J* = 2.9 Hz, 1 H), 7.40 (dd, *J* **=** 9.1, 2.5 Hz, 1 H), 7.30- 7.22 (m, 2 H), 3.86 (s, 3 H); **13C-NMR** (150 MHz, DMSO-*d*6) δ 161.90, 154.77, 149.25, 147.49, 140.39, 134.43, 126.67, 125.93, 125.21, 123.48, 121.62, 118.66, 108.62, 55.96; **HR-MS** *m*/*z* 311.0792 (found), 311.0786 (calc.) [M-H] ; **Purity** (HPLC, 254 nm) 99.0%;

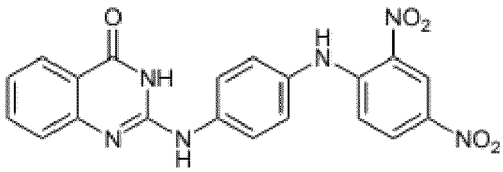

**2-((4-((2,4-Dinitrophenyl)amino)phenyl)amino)-quinazolin-4(3H)-one (31o, BER295)**

**Yield** 71 % (red solid);
**1H-NMR** (400 MHz, DMSO-d6) δ 10.87 (s, 1 H), 10.15 (s, 1 H), 8.90 (d, *J* = 2.7 Hz, 1 H), 8.89 (s, 1 H), 8.86 (s, 1 H), 8.22 (dd, *J* = 9.6, 2.7 Hz, 1 H), 7.98 (d, *J* = 7.8 Hz, 1 H), 7.89 (d, *J* = 8.6 Hz, 2 H), 7.70 - 7.63 (m, 1 H), 7.44 (d, *J* = 8.1 Hz, 1 H), 7.37 (d, *J* = 8.8 Hz, 2 H), 7.29 - 7.22 (m, 1 H), 7.09 (d, *J* = 9.6 Hz, 1 H); **13C-NMR** (100 MHz, DMSO-*d*6) δ 161.55, 149.82, 147.23, 147.12, 137.91, 136.05, 134.48, 131.73, 130.81, 129.75, 126.64, 125.88, 125.35, 123.46, 123.21, 120.16, 118.45, 116.85; **HR-MS** *m*/*z 417.0948* (found), 417.0953 (calc.) [M-H] ; **Purity** (HPLC, 254 nm) 96.5 %;

*Synthetic procedures - N-Methylated 2-(phenylamino)-quinazolin-4(3H)-ones*

**2-(N-Methyl-N-phenylamino)-quinazolin-4(3H)-one (33, BER324)**

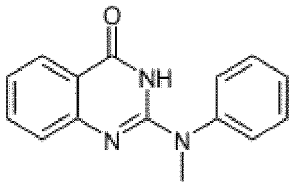

A solution of compound **25** (1.92 g, 10 mmol) and *N*-methylaniline (2.17 ml, 20 mmol) in glacial acetic acid (15 ml) was heated to reflux for 10 h. Upon cooling, water was added dropwise to the solution and a white precipitate started to form. The solution was further diluted with water to a volume of 200 ml and stored in the fridge for 3 h. After filtration, the filtrate was extracted with dichloromethane and ethyl acetate (250 ml each). The organic phases were combined with the filtered precipitate and dried *in vacuo.* Recrystallization from DMF/n-propanol yielded the pure title compound.

**2-(*N*-Methyl-*N*-phenylamino)-quinazolin-4(3*H*)-one (33, BER324)**

**Yield** 88 % (off-white crystals);
**1H-NMR** (400 MHz, DMSO-*d6*) $\delta$ 10.89 (s, 1 H), 7.92 (dd, *J* = 7.9, 1.2 Hz, 1 H), 7.60 (t, *J* = 7.4 Hz, 1 H), 7.45 (t, *J* = 7.7 Hz, 2 H), 7.39 - 7.26 (m, 4 H), 7.22 - 7.16 (m, 1 H), 3.41 (s, 3 H); **13C-NMR** (150 MHz, DMSO-*d6*) $\delta$ 162.43, 150.44, 150.20, 144.03, 134.30, 129.62, 126.35, 125.91, 125.10, 122.60, 117.71, 39.29; **HR-MS** *m/z* 250.0992 (found), 250.0990 (calc.) [M-H] ; **Purity** (HPLC, 254 nm) 98.3 %;

**3- Methyl-2-(phenylamino)-quinazolin-4(3*H*)-one (32, BER341a)**

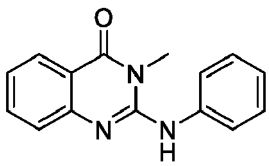

To a stirred suspension of sodium hydride (60 % suspension in mineral oil, 80 mg, 2 mmol) in dry DMF (5 ml) at room temperature (water bath) was added compound **31a** (475 mg, 2 mmol). Then, methyl iodide (125 μl, 2 mmol) was added dropwise and the mixture was stirred for 3 h at 50 °C. The mixture was poured on ice, stirred for 30 min and extracted with ethyl acetate (3 × 150 ml). After evaporation of the solvent *in vacuo,* the crude product was redissolved in ethyl acetate and adsorbed on silica gel (9 g). The pure product was isolated by column chromatography on an Isolera system (100 g silica column) eluting with a hexane/ethyl acetate gradient.

**3-Methyl-2-(phenylamino)-quinazolin-4(3*H*)-one (32, BER341a)**

**Yield** 46 % (colorless solid);
**1H-NMR** (400 MHz, DMSO-d6) $\delta$ 8.65 (s, 1 H), 7.98 (d, *J* = 7.3 Hz, 1 H), 7.74 - 7.65 (m, 2 H), 7.65 - 7.57 (m, 1 H), 7.40 - 7.32 (m, 2 H), 7.27 (d, *J* = 8.1 Hz, 1 H), 7.21 (t, *J* = 7.4 Hz, 1 H), 7.10 (t, *J* = 7.4 Hz, 1 H), 3.61 (s, 3 H); **13C-NMR** (100 MHz, DMSO-*d6*) $\delta$161.75, 148.61, 148.08, 139.30, 134.11, 128.33, 126.38, 124.87, 123.32, 122.73, 122.61, 117.10, 28.79; **HR-MS** *m/z* 252.1124 (found), 252.1131 (calc.) [M+H]+; **Purity** (HPLC, 254 nm) 97.6%;

**3-Methyl-2-(*N*-methyl-*N*-phenylamino)-quinazolin-4(3H)-one (34, BER330a)**

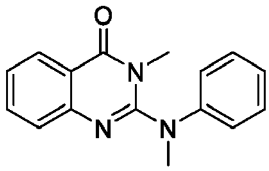

To a stirred suspension of sodium hydride ( 60 % suspension in mineral oil, 352 mg, 8.8 mmol) in dry DMF (15 ml) at room temperature (water bath) was added compound **33** (2.21 g, 8.8 mmol). Then, methyl iodide (560 μl, 9 mmol) was added dropwise and the mixture was stirred for 3 h at 50 °C. The mixture was poured on ice, stirred for 30 min and extracted with ethyl acetate (3 × 150 ml). After evaporation of the solvent *in vacuo,* the crude product was redissolved in ethyl acetate and adsorbed on silica gel (7.5 g). The pure product was isolated by column chromatography on an Isolera system (100 g silica column) eluting with a hexane/ethyl acetate gradient.

**3-Methyl-2-(*N*-methyl-*N*-phenylamino)-quinazolin-4(3*H*)-one (34, BER330a)**

**Yield** 55 % (colorless crystals);
**1H-NMR**(400 MHz, DMSO-d6) δ 8.10 (dd, *J* = 7.9, 1.2 Hz, 1 H), 7.79 - 7.73 (m, 1 H), 7.57 (d, *J* = 8.0 Hz, 1 H), 7.44 - 7.34 (m, 3 H), 7.16 (t, *J* = 7.4 Hz, 1 H), 7.09 (d, *J* = 7.6 Hz, 2 H), 3.41 (s, 3 H), 3.04 (s, 3 H); **13C-NMR** (100 MHz, DMSO-d6) δ 162.86, 152.64, 147.17, 146.87, 134.15, 129.75, 126.23, 125.86, 124.72, 124.10, 121.83, 118.82, 41.59, 31.99; **HR-MS** *m/z* 266.1290 (found), 266.1288 (calc.) [M+H]+; **Purity** (HPLC, 254 nm) 97.9 %;

*Synthetic procedures - Core-modified 2-(phenylamino)-quinazolin-4(3H)-ones*

**7-Nitro-2-(phenylamino)-quinazolin-4(3H)-one (41b, BER413)**

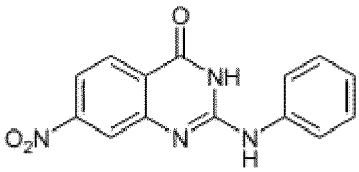

7-Nitro-2-(methylthio)-quinazolin-4(3H)-one **40b** (3.56 g, 15 mmol) and aniline (2.74 ml, 30 mmol) were dissolved in glacial acetic acid and the mixture was refluxed for 18 h. Upon cooling, the reaction was quenched with water resulting in a final volume of 200 ml. After storing the mixture for 3 h in the fridge, the raw product was filtered off, the filtrate was extracted with dichloromethane (2 × 150 ml) and ethyl acetate (150 ml). The crude product and the organic phases were combined and dried *in vacuo.* Recrystallization from DMF/water yielded the pure title compound.

**7-Nitro-2-(phenylamino)-quinazolin-4(3*H*)-one (41b, BER413)**

**Yield** orange solid (69 %);
**1H-NMR** (400 MHz, DMSO-*d*6) δ 11.13 (s, 1 H), 8.85 (s, 1 H), 8.16 (d, *J* = 8.7 Hz, 1 H), 8.10 (d, *J* = 2.2 Hz, 1 H), 7.93 (dd, *J* = 8.7, 2.3 Hz, 1 H), 7.75 (d, *J* = 8.0 Hz, 2 H), 7.38 (t, *J* = 7.8 Hz, 2 H), 7.10 (t, *J* = 7.3 Hz, 1 H); **13C-NMR** (100 MHz, DMSO-*d*6) δ 160.57, 151.42, 150.70, 148.82, 138.33, 128.88, 127.98, 123.08, 122.69, 119.99, 119.81, 116.33; **HR-MS** *m/z* 281.0679 (found), 281.0680 (calc.) [M-H] ; **Purity** (HPLC, 254 nm) 99.7 %;

**6-Nitro-2(phenylamino)-quinazolin-4(3H)-one (41c, BER416)**

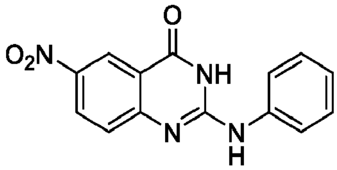

6-Nitro-2(methylthio)-quinazolin-4(3H)-one **40c** (4.75 g, 20 mmol) and aniline (3.65 ml, 40 mmol) were dissolved in glacial acetic acid and the mixture was refluxed for 18 h. Upon cooling, the reaction was quenched with water resulting in a final volume of 200 ml. After storing the mixture for 3 h in the fridge, the raw product was filtered off, dried *in vacuo* and recrystallized from DMF/water.

**6-Nitro-2-(phenylamino)-quinazolin-4(3H)-one (41c, BER416)**

**Yield** yellow solid (79 %);
**1H-NMR** (400 MHz, DMSO-*d*6) $\delta$ 11.22 (s, 1 H), 9.06 (s, 1 H), 8.68 (d, *J* = 2.7 Hz, 1 H), 8.38 (dd, *J* = 9.1, 2.8 Hz, 1 H), 7.73 (d, *J* = 8.0 Hz, 2 H), 7.51 (d, *J* = 9.1 Hz, 1UnH), 7.44 - 7.35 (m, 2 H), 7.17 - 7.08 (m, 1 H); **13C-NMR** (100 MHz, DMSO-d6) $\delta$ 160.77, 155.18, 149.95, 141.85, 137.96, 128.90, 128.53, 126.43, 123.58, 122.31, 120.40, 117.81; **HR-MS** *m*/*z* 281.0679 (found), 281.0680 (calc.) [M-H] ; **Purity** (HPLC, 254 nm) 99.5 %;

**7-Amino-2-(phenylamino)-quinazolin-4(3*H*)-one (42b, BER419)**

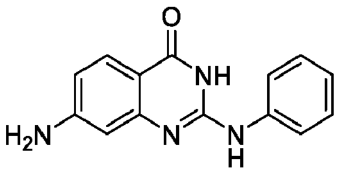

To a suspension of compound **41b** (2.12 g, 7.5 mmol) in ethanol (35 ml), acetic acid (35 ml) and iron filings (1.68 g, 30.0 mmol) were added. The mixture was refluxed for 90 min, then cooled to room temperature. After evaporation of the solvent *in vacuo,* the residue was resuspended in sodium hydroxide solution (5 M, 50 ml) and methanol (50 ml). Leftover iron filings were separated by vacuum filtration, the filtrate was cooled externally with ice, the pH was adjusted to 5 - 6 with conc. hydrochloric acid. After 30 min of stirring, the crude product was filtered off and recrystallized from DMF/water. Since this did not afford pure product, the crystallized solid was subjected to purification by column chromatography on an Isolera system (100 g silica column) eluting with an ethyl acetate/methanol gradient.

**7-Amino-2-(phenylamino)-quinazolin-4(3H)-one (42b, BER419)**

**Yield** pale yellow solid (16 %);
**1H-NMR** (400 MHz, DMSO-d6) $\delta$ 10.22 (s, 1 H), 8.53 (s, 1 H), 7.72 (d, *J* = 7.9 Hz, 2 H), 7.63 (d, *J* = 8.4 Hz, 1 H), 7.40 - 7.29 (m, 2 H), 7.01 (t, *J* = 7.4 Hz, 1 H), 6.51 - 6.42 (m, 2 H), 5.89 (s, 2 H); **13C-NMR** (100 MHz, DMSO-*d*6) $\delta$ 161.01, 154.52, 151.71, 147.25, 139.34, 128.72, 127.16, 121.99, 118.90, 111.63, 107.40, 105.79; **HR-MS** *m*/*z* 253.1082 (found), 253.1084 (calc.) [M+H]+; **Purity** (HPLC, 254 nm) 98.4 %;

**6-Amino-2-(phenylamino)-quinazolin-4(3*H*)-one (42c, BER420)**

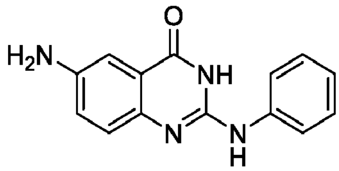

To a suspension of compound **41c** (3.95 g, 14 mmol) in ethanol (65 ml), acetic acid (65 ml) and iron filings (3.13 g, 56 mmol) were added. The mixture was refluxed for 8 h, then cooled to room temperature. After evaporation of the solvent *in vacuo,* the residue was resuspended in sodium hydroxide solution (5 M, 150 ml). Leftover iron filings were separated by vacuum filtration, the filtrate was cooled externally with ice, the pH was adjusted to 5 - 6 with conc. hydrochloric acid. After 30 min of stirring, the crude product was filtered off and recrystallized from DMF/water. Since this did not afford pure product, the crystallized solid was subjected to purification by column chromatography on an Isolera system (340 g silica column) eluting with an ethyl acetate/methanol gradient.

**6-Amino-2-(phenylamino)-quinazolin-4(3H)-one (42c, BER420)**

**Yield** pale yellow solid (30 %);
**1H-NMR** (400 MHz, DMSO-*d*6) $\delta$ 10.55 (s, 1 H), 8.38 (s, 1 H), 7.71 (d, *J* = 8.0 Hz, 2 H), 7.41 - 7.27 (m, 2 H), 7.22 - 7.10 (m, 2 H), 7.03 - 6.94 (m, 2 H), 5.23 (s, 2 H); **13C-NMR** (100 MHz, DMSO-*d*6) $\delta$ 161.54, 145.03, 144.17, 140.60, 139.62, 128.74, 126.06, 122.82, 121.59, 119.06, 118.39, 107.16; **HR-MS** *m*/*z* 251.0938 (found), 251.0938 (calc.) [M-H] ;
**Purity** (HPLC, 254 nm) 95.2%;

Example 1:

High-throughput Screening of Potential Drought Stress Tolerance-inducing Compounds

An improved high-throughput whole-plant bioassay employing *Lemna minor,* based on an assay known in the art, has been developed, which allows for the identification of drought stress tolerance-inducing effects of small molecules. The assay aims at monitoring plant growth under stress conditions, as well as in a subsequent recovery phase with a high level of automatization and accuracy through software-based optical recognition of plant surfaces. Extensive statistical analysis was conducted to define reliable critical effect sizes for small, medium and large effects in plant growth acceleration. Based on the developed assay a compound library was screened containing 119 compounds including candidates for inhibition of human PARP enzymes. A detailed analysis of screening results in relation to the compounds' structural features allowed for the identification of distinct chemical features connected to promising activity and the formulation of a sophisticated lead pharmacophore, partly agreeing with and partly extending the "classical" PARP pharmacophore known from inhibitors of *Hs*PARP-1.

Assay development

The assay known in the art aimed at monitoring growth of *L. minor* plants in 24-well microtiter plates, one plant per well. The utilized STEINBERG medium was equipped with PEG6000 to simulate drought stress over the whole assay period of seven days. Microtiter plates were placed inside an environmental test chamber and plants were grown under constant illumination with semi-automatic measurement of frond areas every 24 h.
Several parameters of the assay were modified to account for effects during recovery from stress and to increase robustness of the statistical model. The assay parameters, including the changes applied, will be discussed in detail.

Assay *duration, stress and recovery phase*

The original assay design allowed for the identification of growth-enhancing effects of compounds under stress conditions only. Possible stabilization of the plants' physiological state under stress conditions, enabling a rapid recovery under non-stress conditions could not be assessed in this setup. To account for these protective effects - resulting in a faster recovery after the stress phase - the assay was split into a stress period of 48 h and a recovery period of 24 h. To ensure a sufficiently high plant growth rate in the shorter time span of the assay, incubation temperature and irradiance were elevated in a range that promotes growth without exposing the plants to additional stress besides the water stress.

*Preparation of growth media*

The assay known in the art sought to impose two different levels of water stress onto the plants by adding PEG6000 to STEINBERG growth medium. -0.2 MPa and -0.3 MPa were chosen as stress levels, corresponding to concentrations of PEG6000 of 180.0 g/l and 211.9 g/l, respectively. Within the scope of this work amounts of PEG6000 were calculated based on the molar relation found in the prior art for 21 °C, resulting in 150 g/l and approximately -0.25 to -0.30 MPa for the standard assay setup.

Starting from the original *in vivo* assay system described in the art, a more robust experimental setup was developed, which allows for the investigation of drought stress tolerance- inducing effects in stress and recovery phase. It was shown that the model plant *L. minor* experiences water stress, despite its natural habitat being water. If equipped with a suitable effect size measure, the assay is able to identify compounds that exhibit both positive and negative effects, while still keeping balance between the number of actives and inactives, thus reducing the share of both false positives and false negatives. In general, this type of assay system can easily be modified and even upscaled to allow application in different ecotoxicological or plant physiological studies, e.g. heavy metal toxicity or nutrient deficiency. By adjusting the composition of the nutrient media, different types of abiological stressors can be imposed onto the plants, while still keeping the benefit of a quick, easy and highly automated evaluation of plant growth.

Example 2:

Assessment of the Biological Activity of Novel Quinazolinones

The growth-enhancing activity towards *Lemna minor* in 2-(arylamino)-quinazolin-4(3H)-ones was confirmed by means of the developed *in vivo* assay, the results of which are shown in Table 1.

Mean screening results of 2-(arylamino)-quinazolin-4(3*H*)-ones

Conclusions regarding the activity of a specific compound and its magnitude shall not be drawn based on single measurements, but rather on a series of repeated experiments to decrease the rate of both false positive and false negative results. For this reason, quinazolin-4(3*H*)-ones were subjected to threefold testing at a concentration of 10 μM in an identical experimental setup as described above. The corresponding effect size classification for mean effect sizes of $n = 3$ experiments, lower and higher numbers, was calculated with the critical values for $n = [2...5]$ replicates given in Table 2.

Table 2: Summary of critical values for the mean effect size assessment depending on number of replicates

| | $n = 2$ | $n = 3$ | $n = 4$ | $n = 5$ |
|---|---|---|---|---|
| Small effect | 1.0 - 1.8 | 0.9 -1.7 | 0.8-1.4 | 0.8 -1.4 |
| Medium effect | 1.8-2.6 | 1.7 - 2.5 | 1.4-2.0 | 1.4 - 2.0 |
| Large effect | > 2.6 | > 2.5 | > 2.0 | > 2.0 |

A subset of the results obtained from repeated testing are shown Fig. 2, ranked according to mean effect sizes. The respective chemical structures and numeric values are given in Table 1 alongside meta results from the initial library screening for identical structures, where available. Firstly, it becomes evident that the ten quinazolinone compounds shown exhibit an effect upon the growth of *L. minor* - three of them achieving a large, three a medium and four compounds a small effect. This fact can be deemed an important proof of concept since the biological activity that was proposed to be inherent for this chemical class could be confirmed in all derivatives tested. Two out of these ten compounds have been investigated earlier as part of the screening library: **C121** and **C122.** Compound **C046** (identical to **C121**) ranked third in the initial screening with an effect size of $\bar{d}_{tot,4} = 2.265 \pm 0.742$, classified a large effect. This effect size was confirmed almost exactly following synthesis of the compound with $\bar{d}_{tot,3} = 2.224 \pm 0.832$, corresponding to a medium effect. It is to be noted that the difference in confidence interval width as well as in effect classification both account for the unequal number of replicate tests. Commercially available compound **C061** originally ranked highest in activity ($\bar{d}_{tot,4} = 2.635 \pm 0.782$) while the identical, in-house synthesized **C122** shows a diminished effect ($\bar{d}_{tot,3} = 2.035 \pm 0.807$) which classifies only as medium. This discrepancy may appear remarkable since mean effect sizes calculated from a number of individual experiments tend to overestimate the true effect with smaller numbers of replicates. However, since both mean effect sizes lie well within the 95 % confidence interval of the respective other, it needs to be concluded that there is no ground for proving statistical difference between the results.

Concentration-dependent activity profiles

So far, all compounds were tested exclusively on 10 μM level to guarantee maximal comparability of results. However, gaining knowledge of the concentration dependency is crucial in assessing the compounds' potency and applicability. Ideally, a compounds' activity, when plotted against the applied concentration on a logarithmic scale, yields a sigmoidal curve starting from low concentration levels without any observable effect to higher concentration levels with a maximal effect (saturation) onto the (biological) system. The shape of the curve, its slope and the location of the half maximal effective concentration ($EC_{50}$) define the therapeutic bandwidth and optimal dose. To probe their concentration dependency of the compounds' potency, the synthesis products were tested over concentration ranges spanning at least four orders of magnitude (factor of 1000) using the previously described *L. minor* assay setup. The number of concentration levels and the maximal concentration applied differ among the tested compounds due to distinct differences in solubility.

*Comparison of model scaffolds*

Compound **C126** represents the structurally least complex 2-(arylamino)-quinazolin-4(3*H*)-one, all other synthesized compounds can be regarded derivatives of this structure. Therefore, the observed activity of this compound serves as benchmark for the evaluation of all other derivatives in terms of growth enhancement:
**C126** was applied over a concentration range of 1 nm to 100 μM as depicted in Fig. 3. As indicated by point size, the number of replicates varied from 1 to 5 for specific concentration levels - the reliability of the estimated effects naturally increases with growing number of replicates, which is expressed through the width of the corresponding confidence intervals on the mean effect sizes. The overall trend of the relation corresponds to the characteristic shape of a sigmoidal curve left of its $EC_{50}$ value. If extrapolated, the curve would reach its inflection point - which marks the compounds' $EC_{50}$ value - and steadily rise towards its maximal value $\overline{d}_{tot,max}$. All tests were initially done without the help of additives (to improve bioavailability, solubility, leaf adhesion or permeation, etc.), thus for the values presented it has to be assumed that the compounds enter the plant purely by passive transport, namely diffusion and osmosis. Both require a large gradient in concentration between compartments, thus explaining the necessity for applying rather high concentrations.

A detailed analysis of the dose-response curve confirms the agreement between the expected and observed concentration dependency of **C126.**

*Electron-rich substituents*

The quinazolinones bearing electron-rich (methoxyl-, dimethylamino-, methylendioxyl-) substituents show a tendency to outperform rather electron-deficient halogen- and nitro-substituted derivatives (Fig. 2). Figs. 5 and 6 inform about the activity profiles of the respective compounds.

At a first glance all six compounds exhibit dose-response relationships that match the expectations gained from the model compound **C126** for the most part. For **C173**, doses of 1 μM to 10 μM begin to enhance the growth of *L. minor* with small to medium, or even large effects until a peak in activity is reached at concentrations of 25 to 100 μM (without additive).

Compounds **C121**, **C122** and **C123** appear as constitutional isomers and show comparable activity profiles, both qualitatively and quantitatively. All three of them exhibit statistically evident growth enhancement in *L. minor* corresponding to small and medium effects at 10 μM level (**C121**: 1.244 ± 0.568; **C122**: 1.455 ± 0.578; **C123:** 1.577 ± 0.606) and large effects at 25 μM (**C121**: 2.756 ± 1.120; **C122:** 3.036 ± 1.178; **C123:** 2.690 ± 1.125) that surpass the model compound **C126.** As concluded from the comparison between **C126** and **C184**, the sterical demand of the 2-substituent seems to influence a compounds' activity much less than its electronic structure. This finding is confirmed by other derivatives: the +*M*-effect of the methoxyl group causes a considerable increase in growth enhancement compared to the model compound **C126**, while its position on the phenyl ring merely finetunes the effect size. Even the bulkier methylendioxyl group in **C128** fails to quench growth enhancement - though activity is clearly decreased in comparison to the methoxyl derivatives, it still matches **C126** in effect size. A limit seems to be reached only with the triple methoxyl substitution in **C173:** while no or only neglectable effects could be observed in the concentration range of 1 nm to 50 μM, a single determination at 100 μM could be judged a large effect. Even if this effect was confirmed in follow-up investigations **C173** would fail to compete with the lower effective doses of its related, less substituted compounds.

Compound **127,** incorporating an electron-donating amino group in the aromatic substituent, shows the highest growth-enhancing activity towards *L. minor* of the whole set (10 μM: 2.937 ± 0.734; 25 μM: 3.654 ± 1.312). Since dialkylamino groups are considered to induce a stronger +*M*-effect than alkoxyl groups, the superiority of **C127** over **C121**, **C122** and **C123** serves as additional evidence for the key importance of the substituents' electronic structure in general and its electron density in particular.

Assessment of growth enhancement under stress and non-stress conditions

As discussed above, stress tolerance-inducing agents likely exhibit plant growth-regulating activity in general, even under normal physiological conditions. With a stress tolerance induction already demonstrated for different 2-(arylamino)-quinazolin-4(3*H*)-ones, the question arises whether a promotion of plant growth could be observed in absence of water stress as well. For the experimental investigation, **C126** was chosen as the model compound for the class of synthesized quinazolinones. The compound was applied to groups of *L. minor* plants at $t_0$ that were subjected to (a) water stress conditions, (b) non-stress conditions, and (c) a combination of a two-day stress and three-day recovery phase. The main experimental parameters as described above were applied with a single modification: 12-well microtiter plates were used instead of 24-well plates, in order to enable plant growth for periods longer than three days without physical limitation by the vessels. This way, a test group could be hosted alongside a DMSO-treated control group in a single plate (both groups with $n = 6$ plants) and monitored for periods of five days. The growth profiles for control and test groups based on logarithmic frond areas are shown in Fig. 14, reflecting the different conditions. Table 3 further informs about the growth rates $\mu$ of control and test groups subjected to the different conditions, which were obtained by linear regression of the mean logarithmic leaf area in the period of days 2 to 4 where maximal growth could be observed. In addition, the calculated frond area doubling time $t_{dbl}$ is given for all groups as more intuitive measure of plant growth.

Table 3: Growth rates and doubling times of *L. minor* under different stress conditions and treatments

| Treatment | | Unstressed | Stressed | 2 d Stress + 3d recovery |
|---|---|---|---|---|
| **C126** | $\mu$ $\frac{1}{d}$ | 0.501 | 0.319 | 0.559 |
| | $t_{dbl}$ [d] | 1.384 | 2.173 | 1.240 |
| DMSO | $\mu$ $\frac{1}{d}$ | 0.425 | 0.232 | 0.454 |
| | $t_{dbl}$ [d] | 1.631 | 2.989 | 1.528 |

The growth profiles for the control groups under stress and non-stress conditions agree with the expected trend. Starting with a decelerated growth within the first 24 h, likely due to physiological adjustment to the "new" environment, both groups pass into an almost linear growth on logarithmic scale which corresponds to an exponential growth in terms of frond area, respectively biomass. The growth rate, which is reflected by the slope of the linear phase, is considerably lower under water stress ($\mu$ = 0.232 1/d) compared to non-stress conditions ($\mu$ = 0.425 1/d). This is also reflected in the respective doubling times of 2.989 d and 1.631 d. In this experimental setup, a doubling time of the unstressed DMSO-treated plants with 1.631 d can be considered the benchmark for optimal growth of *L. minor* which is in agreement with previously reported values. Accordingly, the control group in the stress/recovery experiment shows a delayed growth comparable to the stressed control within the first 48 h and a subsequent acceleration in the recovery phase reflected by an unprecedented shortening in doubling time to 1.528 d. With this acceleration in the recovery period, the stressed control group recovers to a state comparable to the unstressed control. The obtained growth parameters clearly demonstrate that even plants like *L. minor* experience water stress and show distinct physiological adaptations to a change in water potential, despite their natural habitat being water.

The comparison between controls and test groups yields a clear picture at first glance: The test groups treated with 100 µM 2-(phenylamino)-quinazolin-4(3*H*)-one outperform their respective controls in all cases. Under permanent stress conditions the treatment accelerates growth by approximately 38 %, assessed on basis of the growth rates $\mu$ =0.319 1/d (treated) and $\mu$ = 0.232 1/d (control). Although applied only once, the beneficial effect of **C126** is present throughout the whole monitored period, which promises a sustainable long-term effect in a possible field application as well. Interestingly, a positive effect of **C126** is also observed under normal physiological conditions which is reflected by a growth rate increase of 18 % compared to the control. This result suggests that 2-(arylamino)-4(3*H*)-ones indeed exhibit general plant growth-regulating activity, most possibly through interaction with the central regulatory pathways discussed. In agreement, the treatment with **C126** leads to an enhancement in growth by 23 % in the recovery phase, when applied at the beginning of a two-day stress period. It needs to be highlighted that the effect of the chemical treatment is still observable in the recovery phase, although no further application was performed at the shift from stress to recovery conditions, which was achieved by complete exchange of the medium including the chemical agent.

In conclusion, the highest overall growth rate, respectively the lowest doubling time of 1.240 d, could be observed in the recovering plants under treatment with **C126.** The pairwise comparison of treated and non-treated groups under identical conditions reveals the great potential of this class of compounds regarding growth promotion, both under water stress and normal physiological conditions. Regarding the objective of this work, especially the improved production of

biomass during stress situations, as well as the accompanying accelerated recovery seem promising towards a future mitigation of shortfalls in crop production.

The following Table 4 provides a list of more compounds screened within this work.

Table 4: List of compounds screened herein

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 001 | 574 | 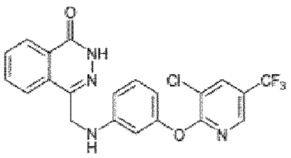 | 0.123 (± 1.136) | -0.468 (± 1.138) | -0.268 (± 1.131) |
| 002 | 575 | 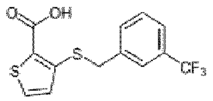 | -0.439 (± 1.137) | 0.066 (± 1.134) | -0.444 (± 1.137) |
| 003 | 576 | 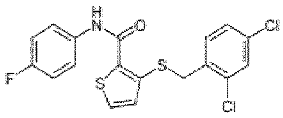 | -0.067 (± 1.130) | -0.784 (± 1.164) | -0.761 (± 1.161) |
| 004 | 577 | 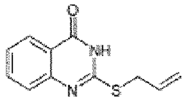 | 0.381 (± 1.153) | -0.430 (± 1.136) | 0.060 (± 1.133) |
| 005 | 578 | 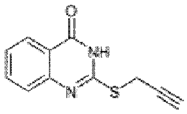 | 0.989 (± 1.231) | -0.386 (± 1.135) | 0.792 (± 1.200) |
| 006 | 579 | 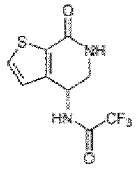 | 0.109 (± 1.135) | 0.368 (± 1.152) | 0.445 (± 1.159) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 007 | 580 | 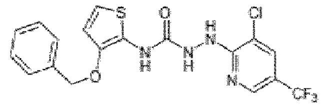 | 0.017 (± 1.132) | 1.227 (± 1.275) | 1.090 (± 1.249) |
| 008 | 581 | 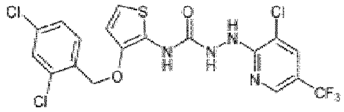 | 0.146 (± 1.137) | 0.860 (± 1.210) | 0.917 (± 1.219) |
| 009 | 582 | 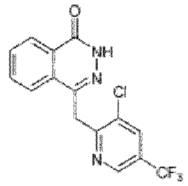 | -0.759 (± 1.161) | 0.339 (± 1.149) | -0.571 (± 1.145) |
| 010 | 583 | 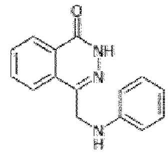 | 0.623 (± 1.178) | 0.664 (± 1.183) | 1.291 (± 1.287) |
| 011 | 584 | 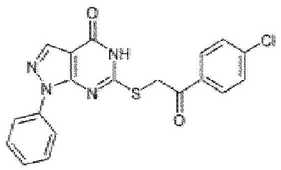 | 1.490 (± 1.330) | 0.707 (± 1.188) | 2.318 (± 1.543) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 012 | 585 | 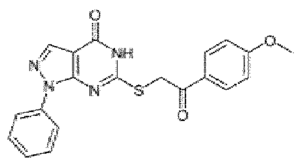 | 0.944 (± 1.224) | 0.498 (± 1.164) | 1.512 (± 1.335) |
| 013 | 586 | 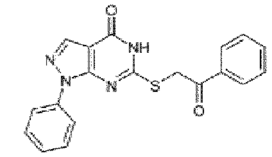 | 0.266 (± 1.144) | -0.293 (± 1.132) | 0.048 (± 1.133) |
| 014 | 587 | 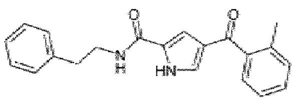 | 0.333 (± 1.149) | -0.870 (± 1.173) | -0.379 (± 1.134) |
| 015 | 588 | 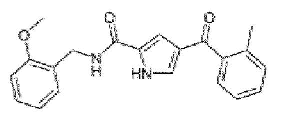 | 0.338 (± 1.149) | -0.682 (± 1.154) | -0.208 (± 1.130) |
| 016 | 589 | 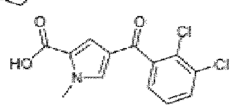 | -1.121 (± 1.208) | -0.353 (± 1.133) | -1.588 (± 1.295) |
| 017 | 590 | 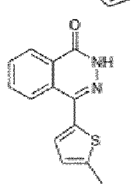 | -1.074 (± 1.201) | -0.404 (± 1.135) | -1.579 (± 1.293) |

Table 4: continued

| BER-C*xxx* | IPB | Structure | $d_{st}(CI_{0.95})$ | $d_{rec}(CI_{0.95})$ | $d_{tot}(CI_{0.95})$ |
|---|---|---|---|---|---|
| 018 | 591 | 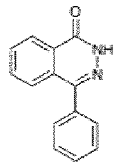 | -0.405 (± 1.135) | -0.183 (± 1.130) | -0.622 (± 1.149) |
| 019 | 592 | 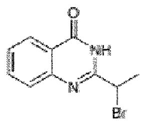 | -0.107 (± 1.130) | 0.256 (± 1.143) | 0.101 (± 1.135) |
| 020 | 593 | 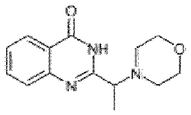 | -0.037 (± 1.131) | 0.791 (± 1.200) | 0.648 (± 1.181) |
| 021 | 594 | 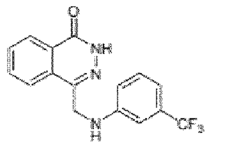 | 0.262 (± 1.144) | 0.115 (± 1.136) | 0.400 (± 1.154) |
| 022 | 595 | 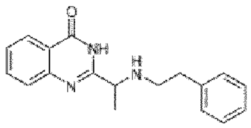 | -0.600 (± 1.147) | 1.366 (± 1.303) | 0.506 (± 1.165) |
| 023 | 596 | 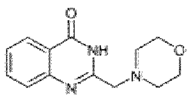 | -0.417 (± 1.136) | 1.869 (± 1.421) | 1.154 (± 1.260) |

Table 4: continued

| BER-C*xxx* | IPB | Structure | $d_{st}(CI_{0.95})$ | $d_{rec}(CI_{0.95})$ | $d_{tot}(CI_{0.95})$ |
|---|---|---|---|---|---|
| 024 | 597 | 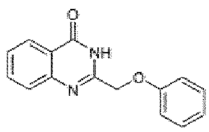 | -0.315 (± 1.132) | 1.620 (± 1.360) | 1.053 (± 1.242) |
| 025 | 598 | 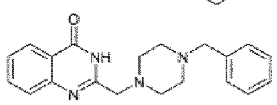 | 0.840 (± 1.207) | -0.125 (± 1.130) | 0.850 (± 1.209) |
| 026 | 599 | 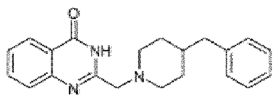 | -0.426 (± 1.136) | 0.359 (± 1.151) | -0.174 (± 1.130) |
| 027 | 600 | 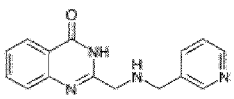 | -0.625 (± 1.149) | 0.372 (± 1.152) | -0.390 (± 1.135) |
| 028 | 601 | 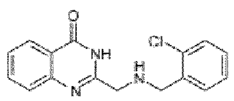 | -0.116 (± 1.130) | 1.388 (± 1.308) | 1.078 (± 1.247) |
| 029 | 602 | 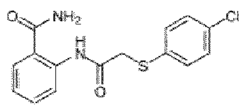 | 0.640 (± 1.180) | 1.543 (± 1.342) | 2.077 (± 1.476) |
| 030 | 603 | 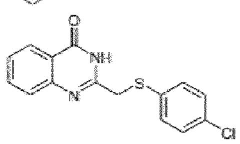 | -0.653 (± 1.151) | 0.679 (± 1.185) | -0.153 (± 1.130) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 031 | 604 | 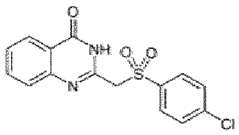 | -0.591 (± 1.146) | 0.309 (± 1.147) | -0.406 (± 1.135) |
| 032 | 605 | 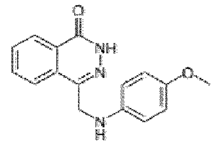 | 0.879 (± 1.213) | 2.019 (± 1.460) | 2.764 (± 1.677) |
| 033 | 606 | 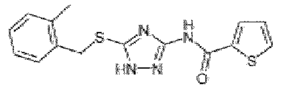 | -0.420 (± 1.136) | 0.364 (± 1.151) | -0.161 (± 1.130) |
| 034 | 607 | 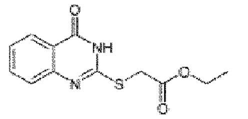 | -1.141 (± 1.211) | 0.640 (± 1.180) | -0.744 (± 1.160) |
| 035 | 608 | 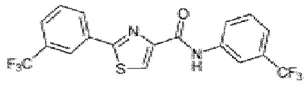 | -0.685 (± 1.154) | 0.331 (± 1.149) | -0.494 (± 1.140) |
| 036 | 609 | 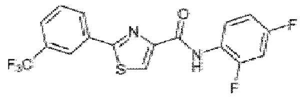 | -0.926 (± 1.180) | 0.286 (± 1.145) | -0.807 (± 1.166) |
| 037 | 610 | 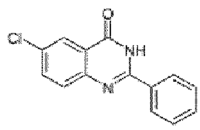 | -0.527 (± 1.142) | 0.828 (± 1.205) | 0.121 (± 1.136) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 039 | 612 | 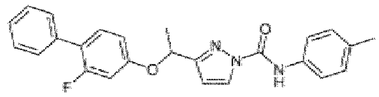 | -0.167 (± 1.130) | 0.559 (± 1.170) | 0.297 (± 1.146) |
| 040 | 613 | 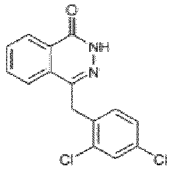 | -0.423 (± 1.136) | 0.630 (± 1.179) | 0.067 (± 1.134) |
| 041 | 614 | 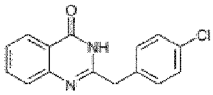 | 0.091 (± 1.135) | 0.780 (± 1.198) | 0.784 (± 1.199) |
| 042 | 615 | 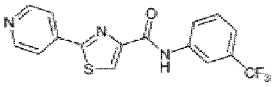 | -0.664 (± 1.152) | 0.307 (± 1.147) | -0.490 (± 1.140) |
| 043 | 616 | 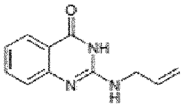 | 0.222 (± 1.141) | 1.416 (± 1.313) | 1.488 (± 1.329) |
| 044 | 617 | 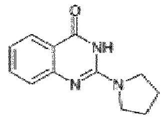 | 0.583 (± 1.173) | 0.441 (± 1.158) | 1.050 (± 1.242) |
| 045 | 618 | 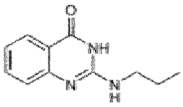 | -0.017 (± 1.131) | 0.624 (± 1.178) | 0.524 (± 1.166) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 046 | 619 | 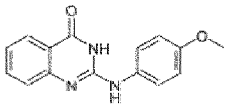 | 1.261 (± 1.281) | 2.181 (± 1.504) | 3.342 (± 1.864) |
| 047 | 620 | 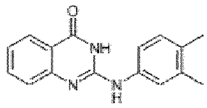 | 0.173 (± 1.138) | 2.939 (± 1.732) | 2.761 (± 1.676) |
| 048 | 621 | 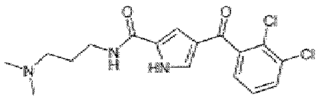 | -0.300 (± 1.132) | 1.645 (± 1.366) | 1.092 (± 1.249) |
| 049 | 622 | 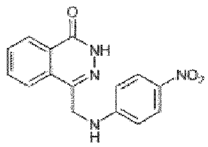 | -0.693 (± 1.155) | 0.546 (± 1.169) | -0.316 (± 1.132) |
| 050 | 623 | 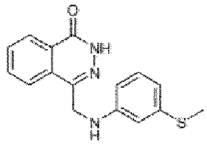 | 0.346 (± 1.150) | 1.543 (± 1.342) | 1.741 (± 1.389) |
| 051 | 624 | 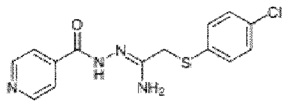 | -0.299 (± 1.132) | 1.183 (± 1.266) | 0.690 (± 1.186) |
| 052 | 625 | 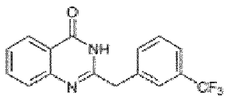 | 0.107 (± 1.135) | 0.167 (± 1.138) | 0.268 (± 1.144) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 053 | 626 | 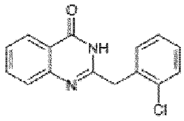 | 0.496 (± 1.164) | 0.436 (± 1.158) | 0.947 (± 1.224) |
| 054 | 627 | 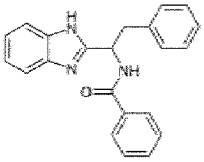 | 0.522 (± 1.166) | 0.800 (± 1.201) | 1.294 (± 1.288) |
| 055 | 628 | 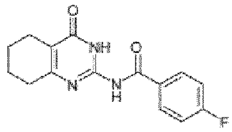 | -0.354 (± 1.133) | -0.474 (± 1.139) | -0.817 (± 1.167) |
| 056 | 629 | 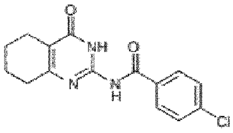 | 0.037 (± 1.133) | -0.633 (± 1.149) | -0.510 (± 1.141) |
| 057 | 630 | 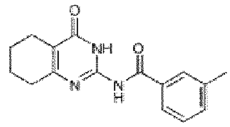 | -0.627 (± 1.149) | -0.348 (± 1.133) | -1.020 (± 1.193) |
| 058 | 631 | 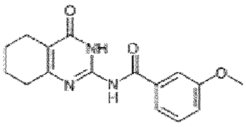 | -0.330 (± 1.133) | -0.050 (± 1.131) | -0.420 (± 1.136) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 059 | 632 | 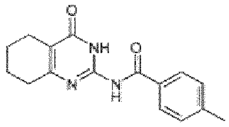 | -0.410 (± 1.136) | 0.315 (± 1.147) | -0.194 (± 1.130) |
| 060 | 633 | 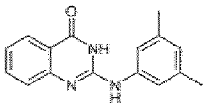 | 0.865 (± 1.211) | 0.966 (± 1.227) | 1.830 (± 1.411) |
| 061 | 634 | 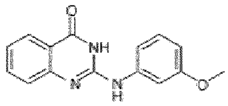 | 1.422 (± 1.315) | 0.611 (± 1.176) | 2.157 (± 1.498) |
| 062 | 635 | 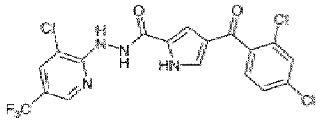 | 0.263 (± 1.144) | -0.448 (± 1.137) | -0.091 (± 1.130) |
| 063 | 636 | 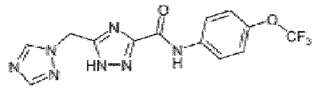 | 0.302 (± 1.146) | -0.014 (± 1.131) | 0.333 (± 1.149) |
| 064 | 637 | 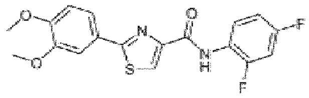 | -0.513 (± 1.141) | 1.203 (± 1.270) | 0.463 (± 1.160) |
| 065 | 638 | 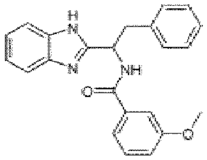 | 0.114 (± 1.135) | 1.400 (± 1.310) | 1.352 (± 1.300) |

Table 4: continued

| BER-C*xxx* | IPB | Structure | $d_{st}$ $(CI_{0.95})$ | $d_{rec}$ $(CI_{0.95})$ | $d_{tot}$ $(CI_{0.95})$ |
|---|---|---|---|---|---|
| 066 | 639 | 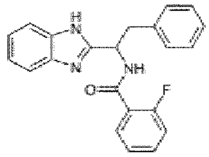 | -0.719 (± 1.157) | 1.262 (± 1.282) | 0.280 (± 1.145) |
| 067 | 640 | 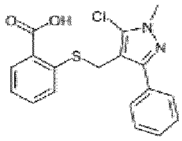 | -0.165 (± 1.130) | 0.566 (± 1.171) | 0.305 (± 1.147) |
| 068 | 641 | 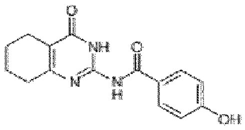 | -0.190 (± 1.130) | 0.443 (± 1.158) | 0.169 (± 1.138) |
| 069 | 642 | 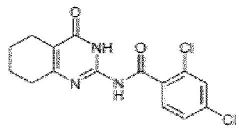 | -0.659 (± 1.152) | 0.053 (± 1.133) | -0.706 (± 1.156) |
| 070 | 643 | 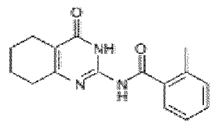 | -0.037 (± 1.131) | -0.214 (± 1.130) | -0.229 (± 1.130) |
| 071 | 644 | 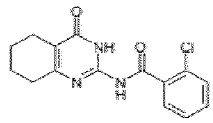 | 0.922 (± 1.220) | 0.694 (± 1.187) | 1.658 (± 1.369) |

Table 4: continued

| BER-C*xxx* | IPB | Structure | $d_{st}$ $(CI_{0.95})$ | $d_{rec}$ $(CI_{0.95})$ | $d_{tot}$ $(CI_{0.95})$ |
|---|---|---|---|---|---|
| 072 | 645 | 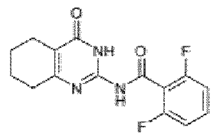 | -0.161 (± 1.130) | -0.515 (± 1.141) | -0.634 (± 1.150) |
| 073 | 646 | 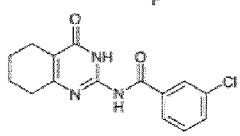 | -0.006 (± 1.131) | 0.227 (± 1.141) | 0.191 (± 1.139) |
| 074 | 647 | 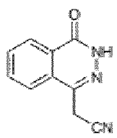 | 0.277 (± 1.145) | 0.618 (± 1.177) | 0.856 (± 1.210) |
| 075 | 648 | 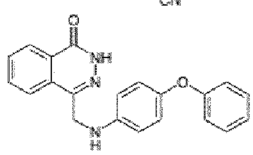 | 0.681 (± 1.185) | 0.940 (± 1.223) | 1.597 (± 1.354) |
| 076 | 649 | 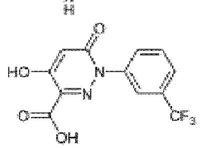 | 0.963 (± 1.227) | 0.200 (± 1.140) | 1.273 (± 1.284) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 077 | 650 | 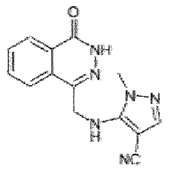 | -0.469 (± 1.138) | 0.836 (± 1.207) | 0.194 (± 1.139) |
| 078 | 651 | 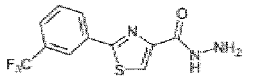 | 1.134 (± 1.257) | 0.876 (± 1.213) | 2.058 (± 1.471) |
| 079 | 652 | 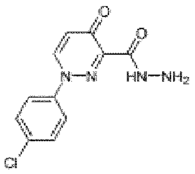 | 0.805 (± 1.202) | 0.659 (± 1.182) | 1.493 (± 1.331) |
| 080 | 653 | 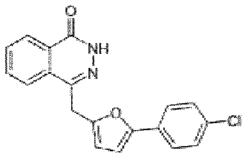 | -0.269 (± 1.131) | 0.349 (± 1.150) | -0.003 (± 1.132) |
| 081 | 654 | 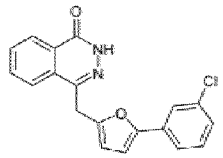 | -0.262 (± 1.131) | 0.602 (± 1.175) | 0.227 (± 1.141) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 092 | 665 | 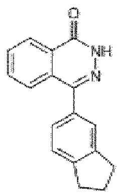 | 0.390 (± 1.154) | 1.658 (± 1.369) | 1.892 (± 1.427) |
| 093 | 666 | 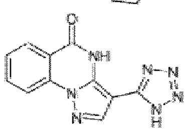 | -0.563 (± 1.144) | 0.761 (± 1.196) | 0.020 (± 1.132) |
| 094 | 667 | 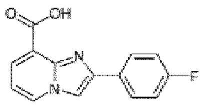 | 0.107 (± 1.135) | 1.040 (± 1.240) | 1.029 (± 1.238) |
| 095 | 668 | 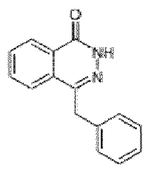 | 0.764 (± 1.196) | 1.045 (± 1.241) | 1.784 (± 1.399) |
| 096 | 669 | 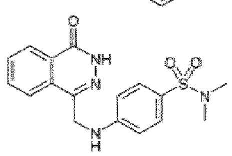 | 0.451 (± 1.159) | 1.836 (± 1.412) | 2.117 (± 1.487) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 097 | 670 | 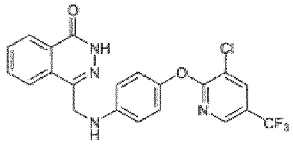 | -0.121 (± 1.130) | 0.868 (± 1.211) | 0.619 (± 1.177) |
| 098 | 671 | 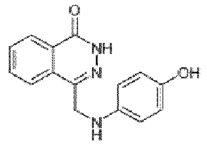 | 0.219 (± 1.141) | 0.158 (± 1.138) | 0.388 (± 1.153) |
| 099 | 672 | 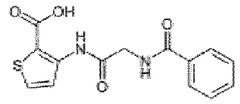 | -0.371 (± 1.134) | 0.008 (± 1.132) | -0.417 (± 1.136) |
| 100 | 673 | 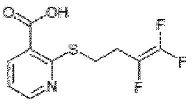 | -1.453 (± 1.267) | 0.215 (± 1.141) | -1.472 (± 1.271) |
| 101 | 674 | 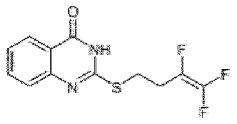 | -1.025 (± 1.193) | 1.232 (± 1.276) | -0.095 (± 1.130) |
| 102 | 675 | 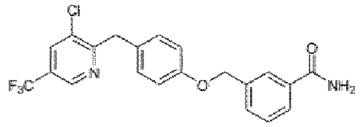 | -0.898 (± 1.177) | -0.068 (± 1.130) | -1.085 (± 1.202) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 103 | 676 | 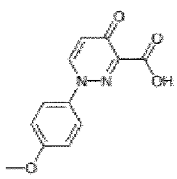 | -0.235 (± 1.130) | 0.509 (± 1.165) | 0.176 (± 1.138) |
| 104 | 677 | 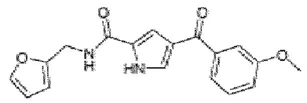 | -0.258 (± 1.131) | 0.858 (± 1.210) | 0.454 (± 1.159) |
| 105 | 678 | 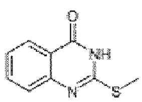 | 0.004 (± 1.132) | -0.051 (± 1.131) | -0.040 (± 1.131) |
| 106 | 679 | 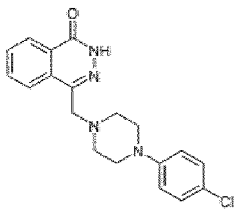 | -0.162 (± 1.130) | -0.334 (± 1.133) | -0.477 (± 1.139) |
| 107 | 680 | 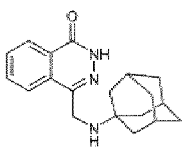 | -0.520 (± 1.141) | 0.471 (± 1.161) | -0.183 (± 1.130) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 108 | 681 | 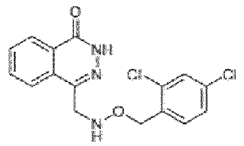 | -0.312 (± 1.132) | 0.362 (± 1.151) | -0.041 (± 1.131) |
| 109 | 682 | 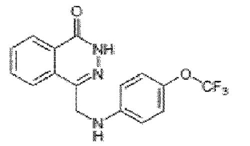 | 0.209 (± 1.140) | -1.074 (± 1.201) | -0.697 (± 1.155) |
| 110 | 683 | 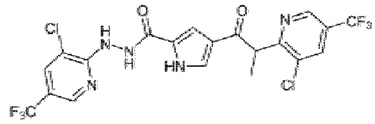 | -0.298 (± 1.132) | -0.799 (± 1.165) | -1.036 (± 1.195) |
| 111 | 684 | 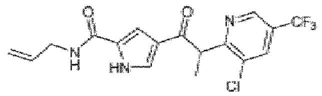 | 0.623 (± 1.178) | -0.377 (± 1.134) | 0.382 (± 1.153) |
| 112 | 685 | 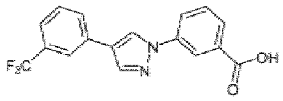 | -0.288 (± 1.131) | 1.008 (± 1.234) | 0.551 (± 1.169) |
| 113 | 686 | 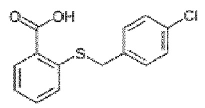 | -1.350 (± 1.247) | 0.919 (± 1.220) | -0.739 (± 1.159) |
| 114 | 687 | 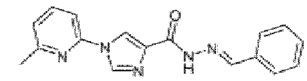 | -0.747 (± 1.160) | 0.432 (± 1.157) | -0.477 (± 1.139) |

Table 4: continued

| BER-C*xxx* | IPB | Structure | $d_{st}$ ($CI_{0.95}$) | $d_{rec}$ ($CI_{0.95}$) | $d_{tot}$ ($CI_{0.95}$) |
|---|---|---|---|---|---|
| 115 | 688 | 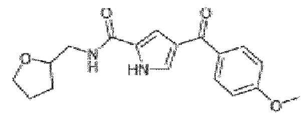 | 1.249 (± 1.279) | 0.217 (± 1.141) | 1.616 (± 1.359) |
| 116 | 689 | 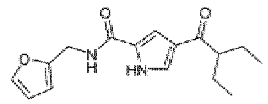 | 0.562 (± 1.171) | 0.309 (± 1.147) | 0.912 (± 1.218) |
| 117 | 690 | 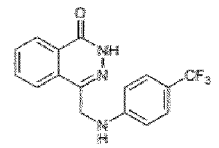 | 0.891 (± 1.215) | -0.087 (± 1.130) | 0.942 (± 1.223) |
| 118 | 691 | 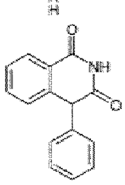 | -0.968 (± 1.186) | 0.404 (± 1.155) | -0.753 (± 1.160) |
| 119 | 692 | 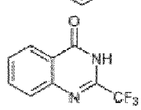 | -1.749 (± 1.331) | 0.261 (± 1.144) | -1.770 (± 1.336) |
| 120 | 693 | 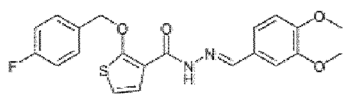 | -0.533 (± 1.142) | 1.343 (± 1.298) | 0.563 (± 1.171) |

## Claims

**1.** Use of a compound according to Formula (I) as a phytoeffector:

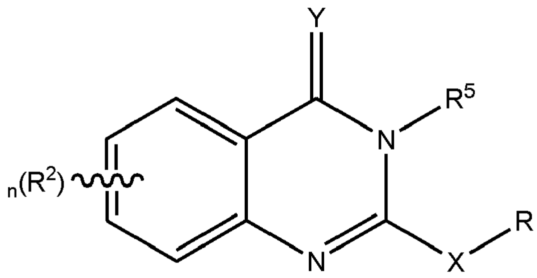

(I)

wherein

Y is O, N-$NR^3_2$, N-$OR^3$, or $NR^3$,
X is $NR^4$, S, SO, $SO_2$, O, or $CH_2$,
R is H, alkyl, cycloalkyl, unsubstituted or substituted aryl, or unsubstituted or substituted alkaryl; or, if X stands for $CH_2$, then, R can also be alkoxy, unsubstituted or substituted oxyaryl, or morpholinyl,
$R^2$ is H, halogen, nitro, unsubstituted or substituted amino, alkyl, alkoxy, unsubstituted or substituted alkynyl or unsubstituted or substituted aryl; or $R^2$ stands for one or more annellated aromatic rings to form a naphthalene, anthracene, or phenanthrene moiety,
$R^3$ is H, alkyl, alkaryl, unsubstituted or substituted heteroaryl or unsubstituted or substituted aryl,
$R^4$ is H, alkyl, alkaryl, unsubstituted or substituted heteroaryl or unsubstituted or substituted aryl,
$R^5$ is H, alkyl, alkaryl, unsubstituted or substituted heteroaryl or unsubstituted or substituted aryl, and
n is 0, 1, 2, 3, or 4,
where the aforementioned substituent(s) can be selected from the group consisting of halogen, alkyl, alkoxy, aryloxy, trialkylsilyl, nitro, unsubstituted or substituted amino including aminoheterocycles.

**2.** The use according to claim 1, where in the compound according to Formula (I), Y is O.

**3.** The use according to claim 1 or claim 2, where in the compound according to Formula (I), X is NH.

**4.** The use according to any one of claims 1 to 3, where in the compound according to Formula (I), R is aryl.

**5.** The use according to claim 4, wherein the aryl is an electron-rich aryl.

**6.** The use according to claim 1, wherein the compound according to Formula (I) is selected from the group consisting of:

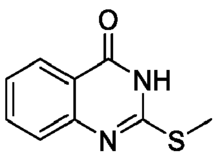,

(Compound BER384 (QSP265, GOL102))

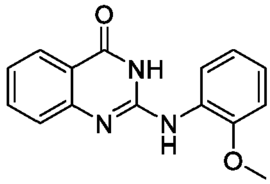

(Compound C123 (BER287))

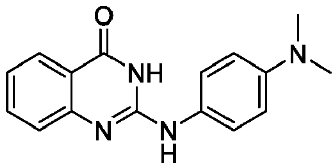

,

(Compound C127 (BER263, BER294)

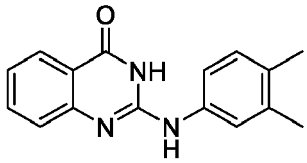

,

(Compound C047 (QSP207))

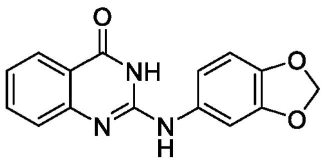

,

(Compound C128 (BER275, BER299)

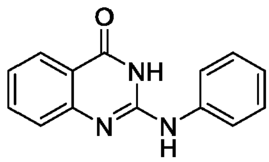

,

(Compound C126 (BER238))

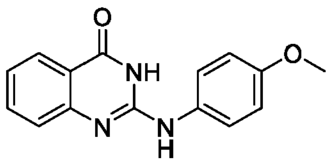

,

(Compound C121 (BER248, QSP206)

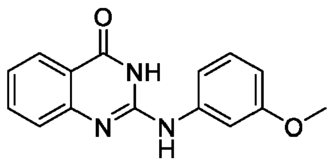

,

(Compound C122 (BER278, QSP221))

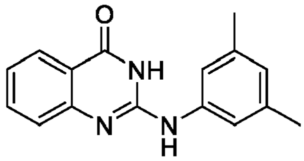

,

(Compound C060 (QSP220))

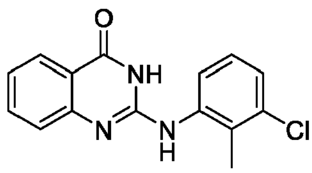

,

(Compound C130 (BER306))

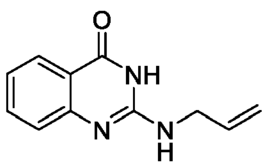

,

(Compound C043 (QSP203))

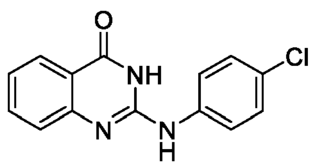

,

(Compound C124 (BER276, BER304))

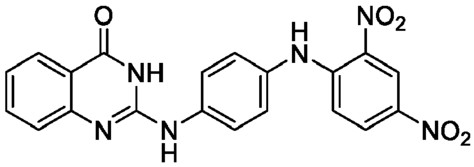
,

(Compound C172 (BER295))

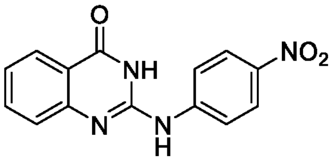
,

(Compound C129 (BER262, BER288))

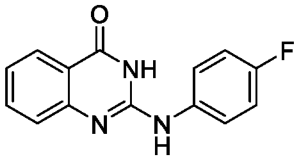
,

(Compound C125 (BER261, BER305))

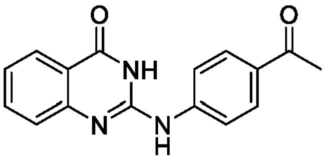
,

(Compound C178 (BER338))

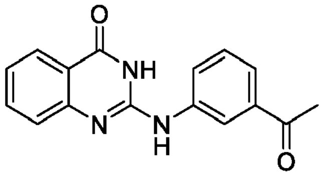
,

(Compound C179 (BER329, BER389))

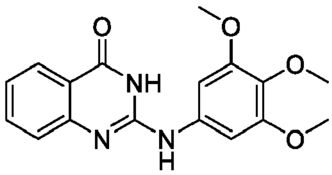
,

(Compound C173 (BER300))

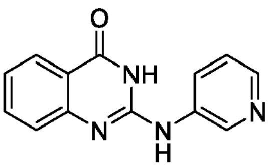
,

(Compound GOL192)

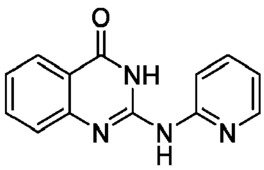
,

(Compound GOL193)

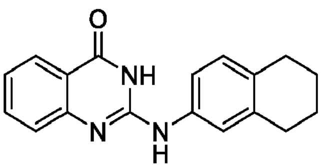
,

(Compound GOL194)

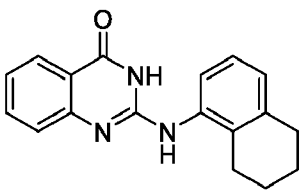
,

(Compound GOL195)

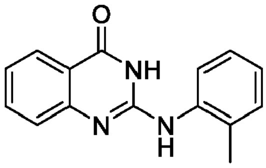
,

(Compound GOL298)

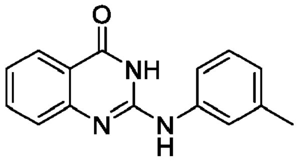
,

(Compound GOL299)

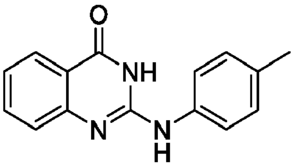
,

(Compound GOL300)

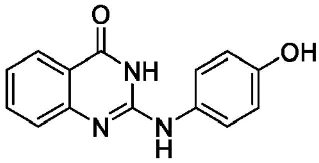
,

(Compound GOL321)

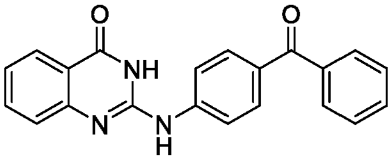
,

(Compound GOL322)

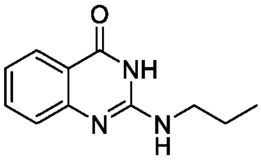
,

(Compound C045 (QSP205))

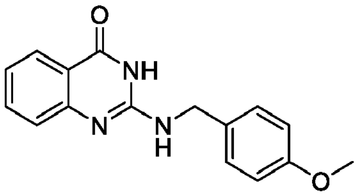
,

(Compound GOL318)

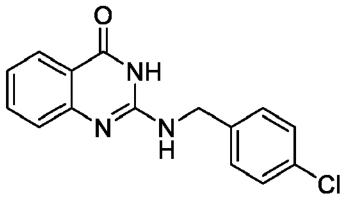
,

(Compound GOL319)

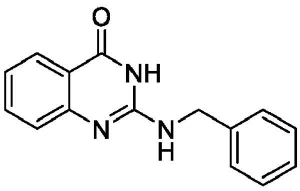
,

(Compound GOL323)

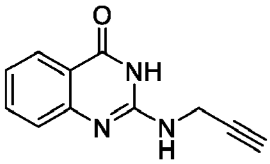
,

(Compound GOL332)

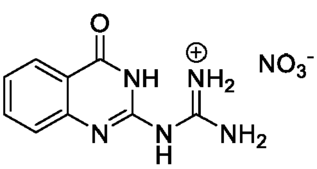
,

(Compound BER255)

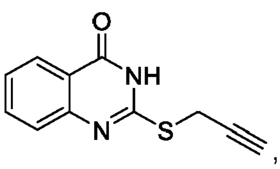
,

(Compound C005 (GOL331, QSP165))

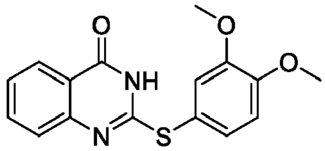,

(Compound GOL187)

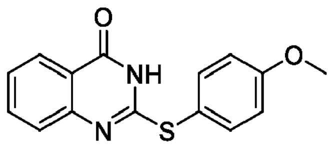,

(Compound GOL188)

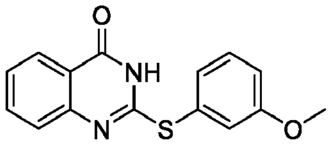,

(Compound GOL189)

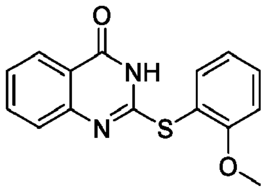,

(Compound GOL190)

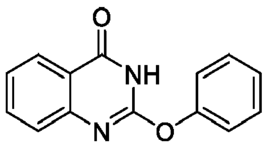,

(Compound GOL407)

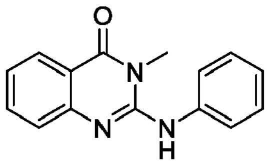

(Compound C176 (BER341))

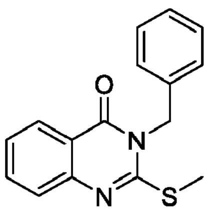

(Compound BER356)

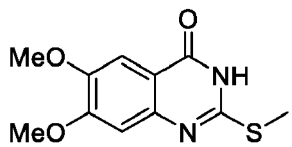,

(Compound BER365)

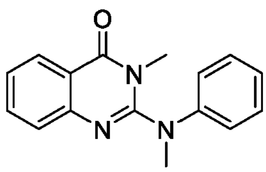,

(Compound C177 (BER330))

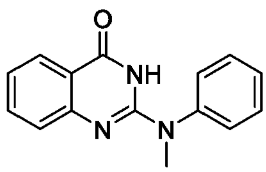,

(Compound C175 (BER308))

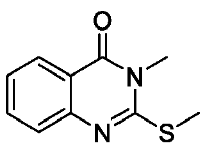,

(Compound BER319)

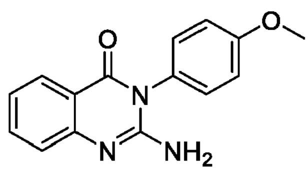,

(Compound BER220)

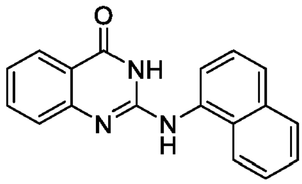
,

(Compound BER321)

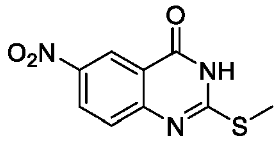
,

(Compound BER407)

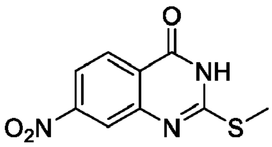
,

(Compound BER408)

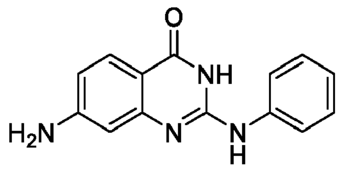
,

(Compound C182 (BER419))

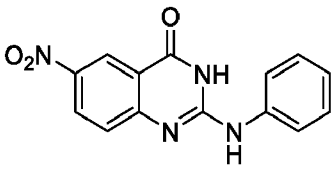
,

(Compound C181 (BER416))

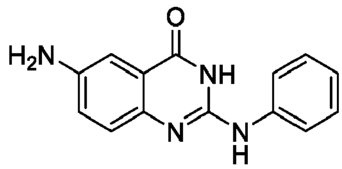
,

(Compound C183 (BER420))

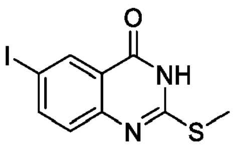
,

(Compound GOL374)

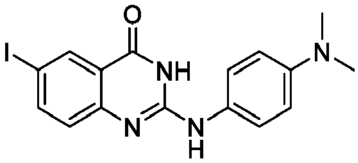
,

(Compound GOL375)

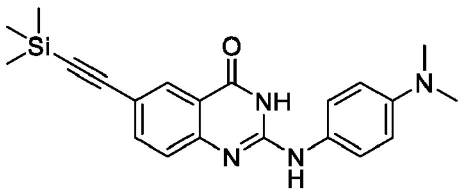
,

(Compound GOL385)

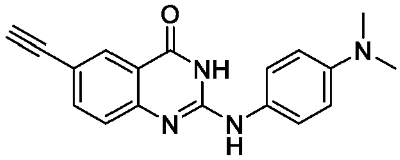
,

(Compound GOL389)

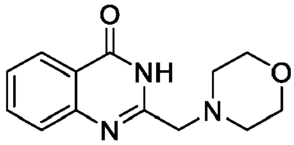

(Compound C023 (QSP183))

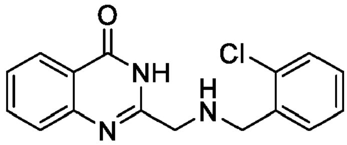,
(Compound C028 (QSP188))

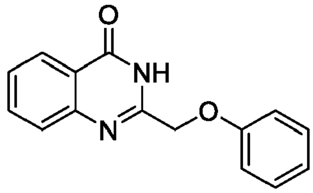.
(Compound C024 (QSP184))

**7.** The use according to claim 1, wherein the compound according to Formula (I) is selected from the group consisting of:

Compound C123 (BER287),
Compound C127 (BER263, BER294),
Compound C047 (QSP207),
Compound C128 (BER257, BER299),
Compound GOL389,
Compound BER384 (QSP265, GOL102),
Compound C126 (BER238),
Compound C121 (BER248, QSP206),
Compound C122 (BER278, QSP221),
Compound C060 (QSP220),
Compound C130 (BER306), and
Compound C043 (QSP203);
preferably from the group consisting of:

Compound C123 (BER287),
Compound C127 (BER263, BER294),
Compound C047 (QSP207),
Compound C128 (BER257, BER299),
Compound GOL389,
Compound BER384 (QSP265, GOL102),
Compound C126 (BER238),
Compound C121 (BER248, QSP206), and
Compound C122 (BER278, QSP221);
more preferably from the group consisting of:

Compound C123 (BER287),
Compound C127 (BER263, BER294),
Compound C047 (QSP207),
Compound C128 (BER257, BER299),
Compound GOL389, and
Compound BER384 (QSP265, GOL102);
yet more preferably from the group consisting of
Compound C123 (BER287), and
Compound BER384 (QSP265, GOL102).

**8.** The use according to claim 1, wherein the compound according to Formula (I) is Compound C123 (BER287).

**9.** The use according to claim 1, wherein the compound according to Formula (I) is selected from the group consisting of:

Compound GOL374, Compound GOL193, and Compound GOL190,
preferably wherein the compound is Compound GOL374.

**10.** The use according to any one of claims 1 to 9, wherein the phytoeffector provides one or more of the following effects:

(i) increasing plant growth,
(ii) increasing plant health,

(iii) increasing plant biomass,
(iv) increasing plant yield,
(v) decreasing plant growth,
(vi) inhibiting plant growth,
(vii) abolishing pant growth,
(viii) killing the plant, and
(vii) inhibiting plant PARP enzymes.

**11.** The use according to claim 10, wherein the plant is undergoing abiotic stress.

**12.** The use according to claim 11, wherein the abiotic stress is drought stress.

**13.** A compound, selected from the group consisting of

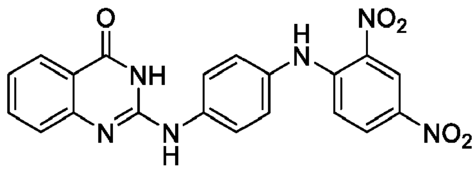
,

(Compound C172 (BER295))

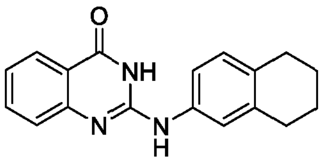
,

(Compound GOL194)

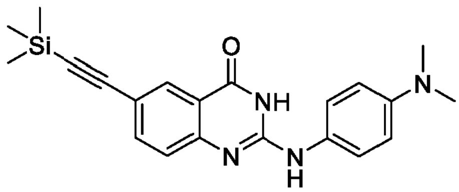
,

(Compound GOL385)

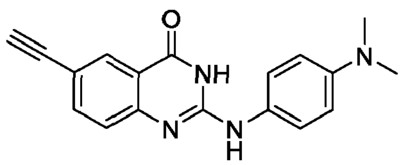
.

(Compound GOL389)

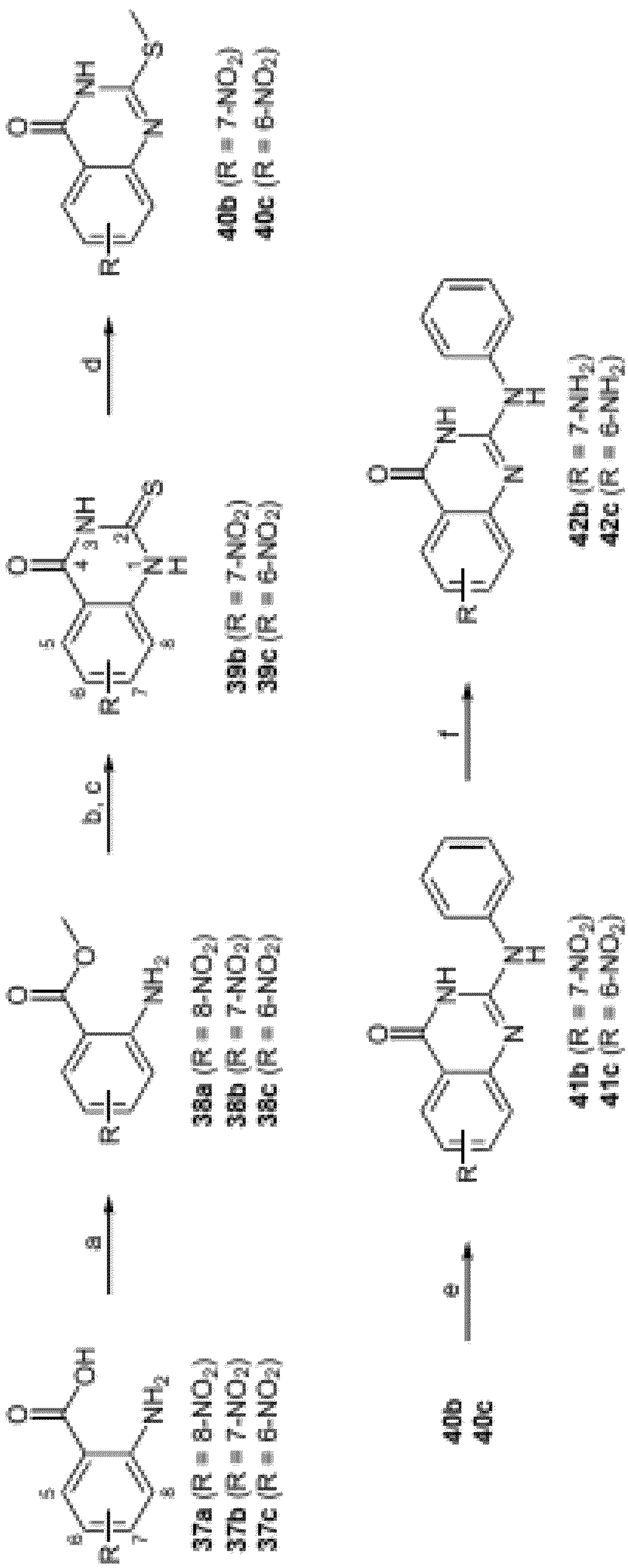


37a (R = 8-NO$_2$)
37b (R = 7-NO$_2$)
37c (R = 6-NO$_2$)

38a (R = 8-NO$_2$)
38b (R = 7-NO$_2$)
38c (R = 6-NO$_2$)

39b (R = 7-NO$_2$)
39c (R = 6-NO$_2$)

40b (R = 7-NO$_2$)
40c (R = 6-NO$_2$)

40b
40c

41b (R = 7-NO$_2$)
41c (R = 6-NO$_2$)

42b (R = 7-NH$_2$)
42c (R = 6-NH$_2$)

Figure 1

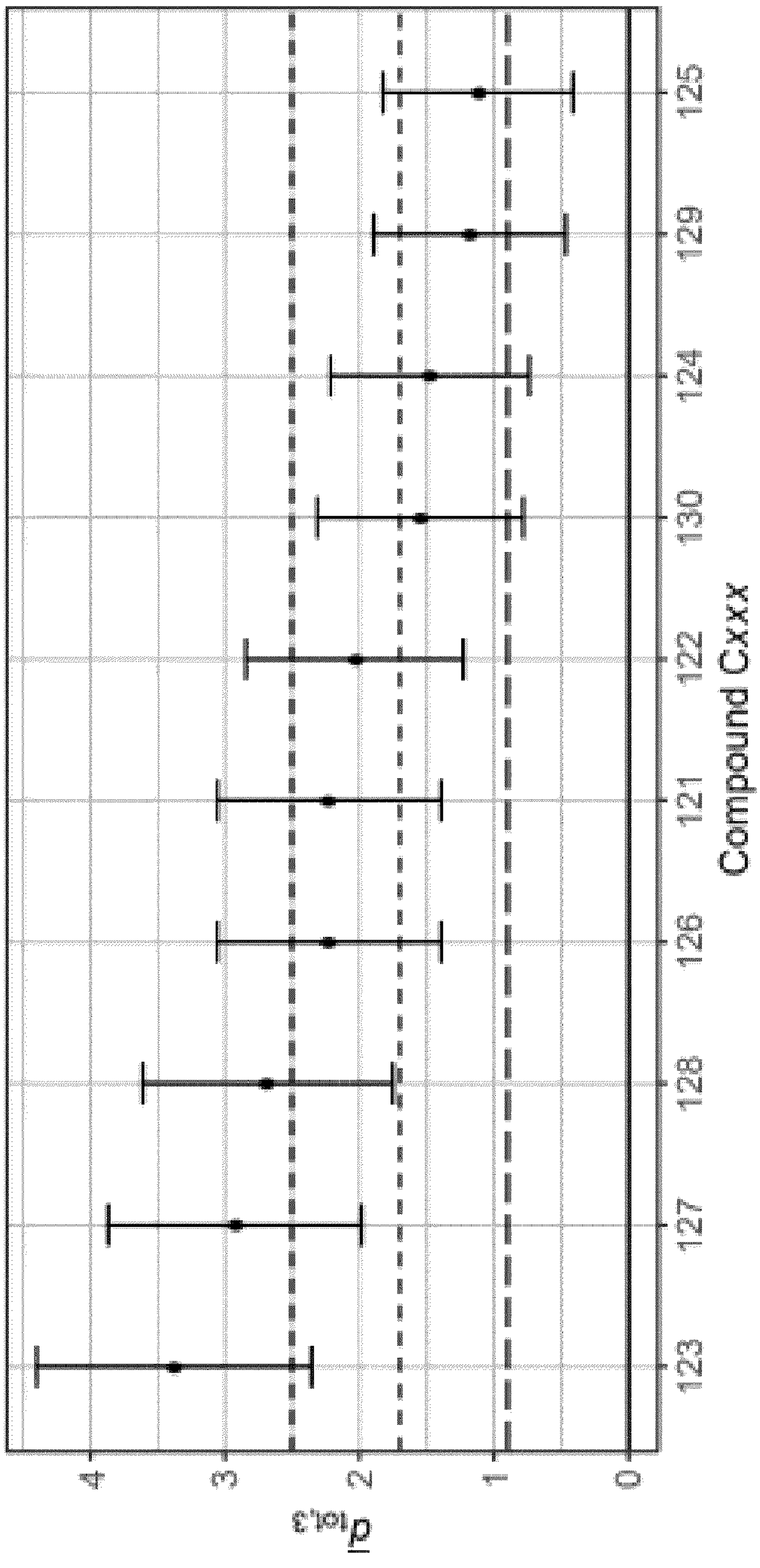


Figure 2

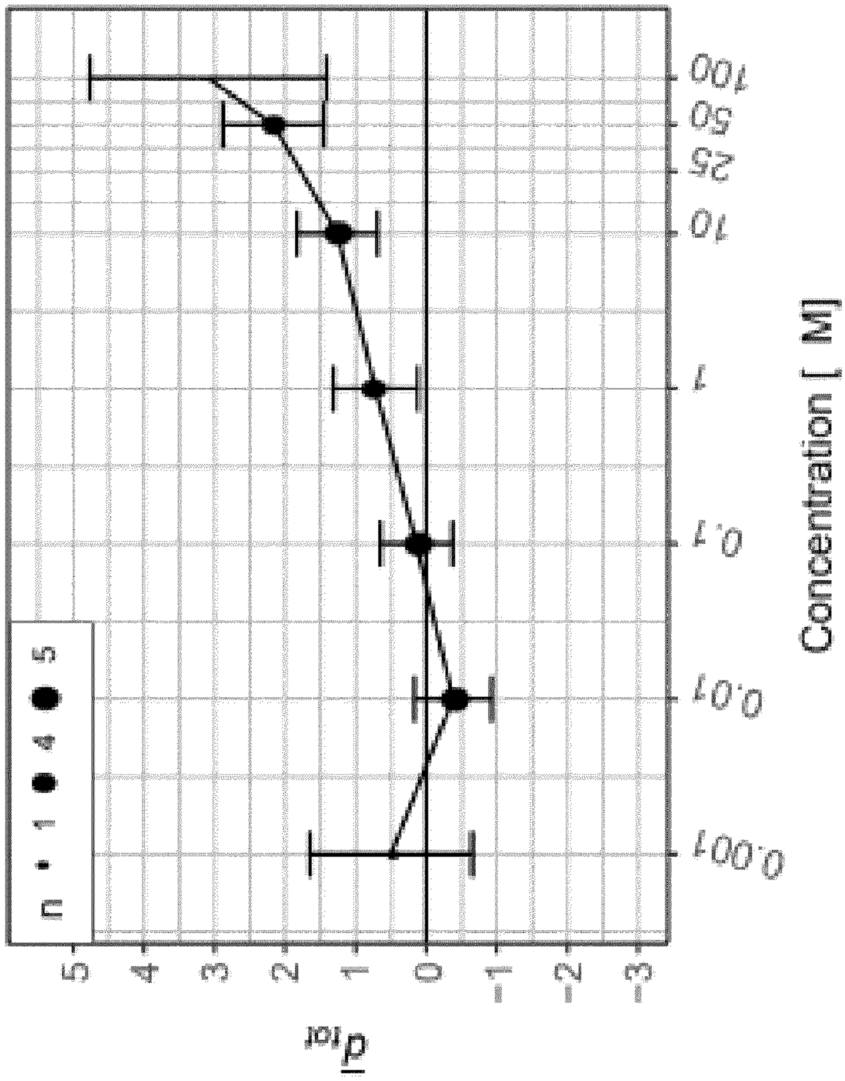


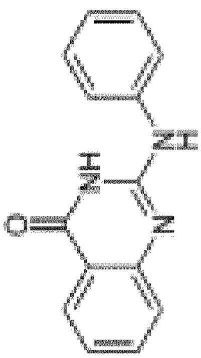

C126

Figure 3

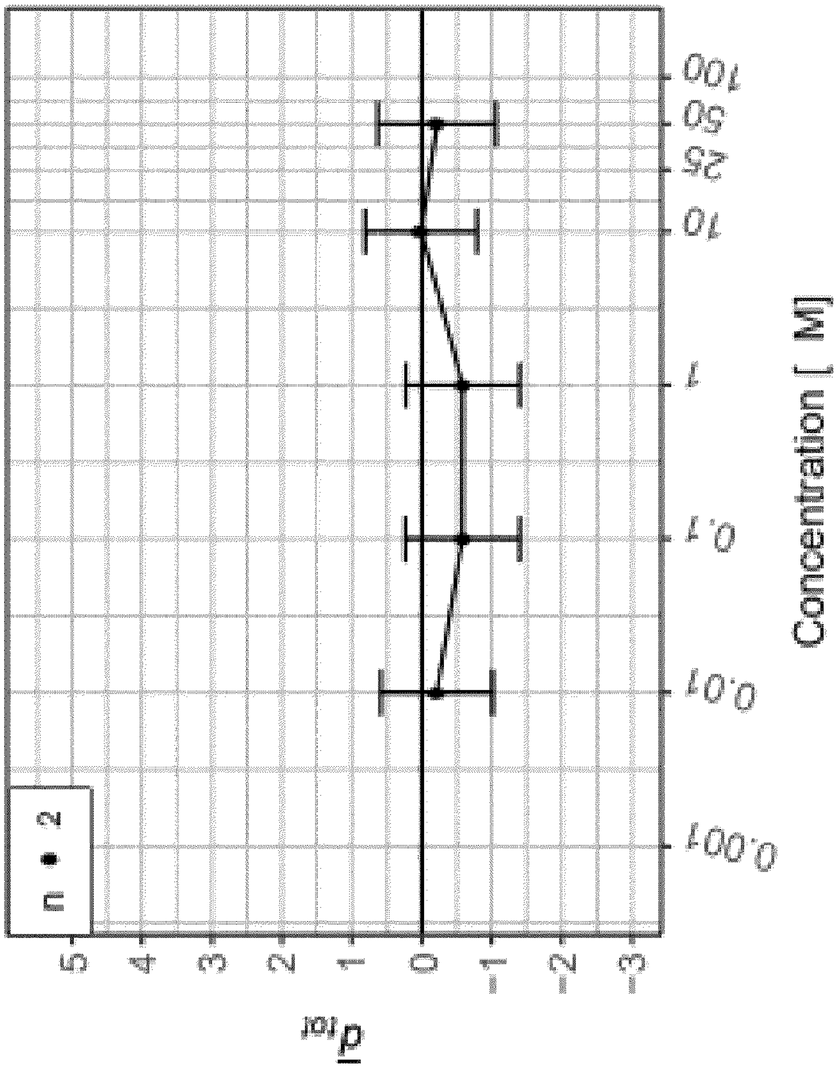


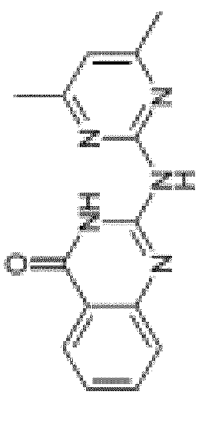

C184

Figure 4

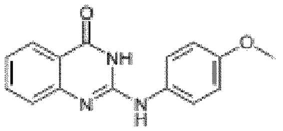

C121

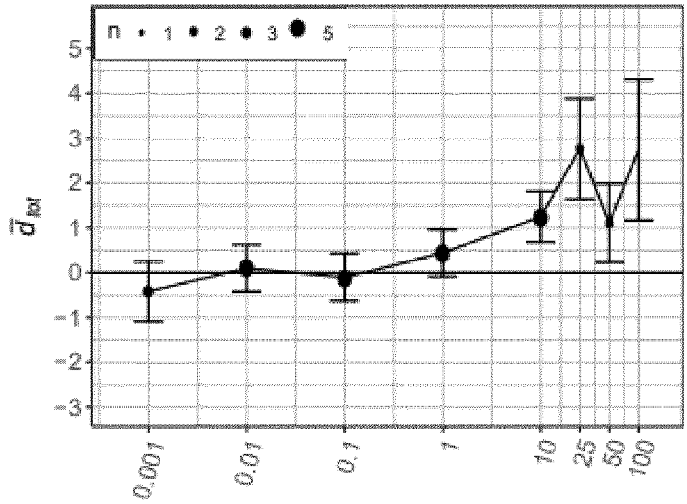


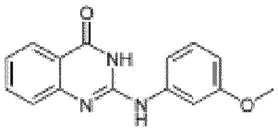

C122

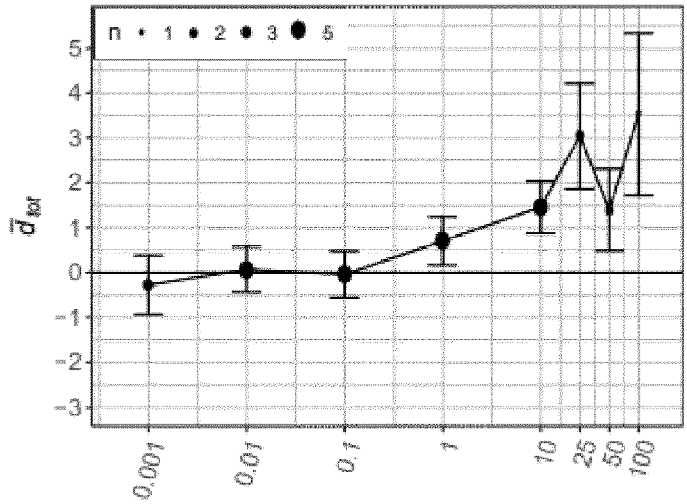


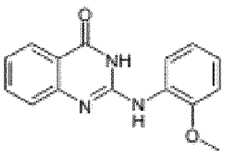

C123

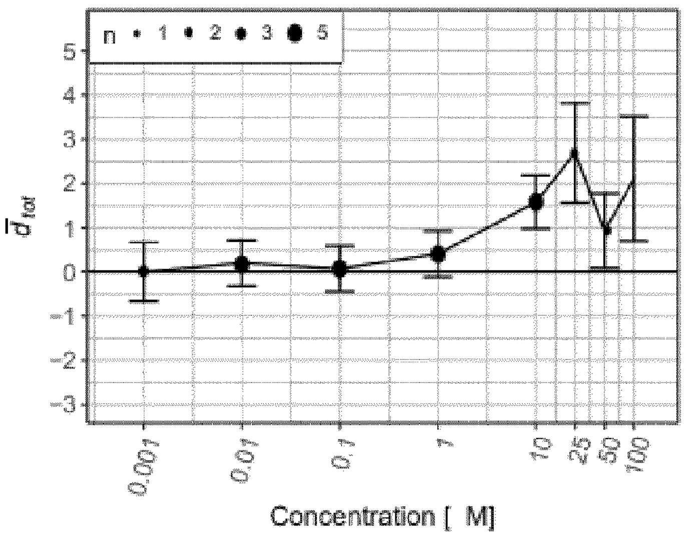


Figure 5

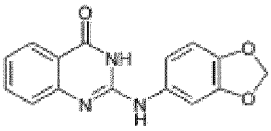

C128

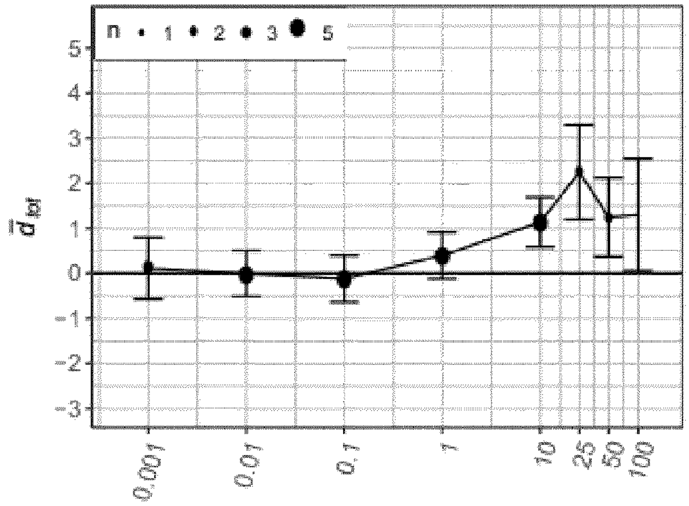


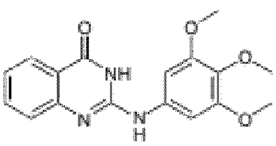

C173

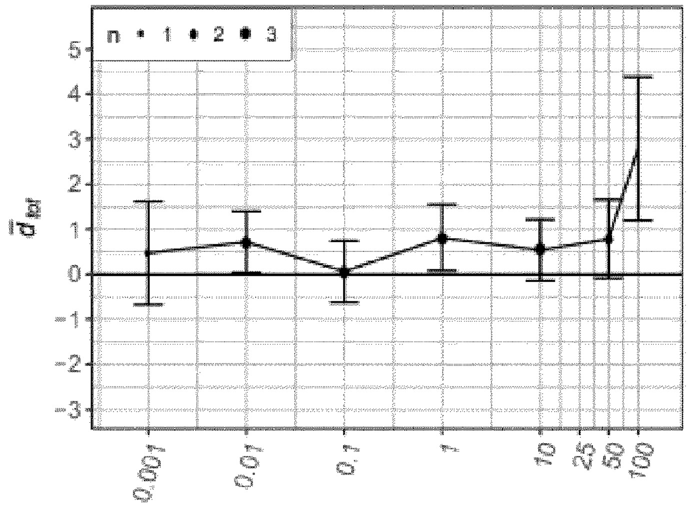


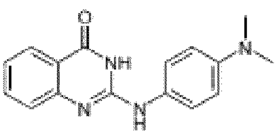

C127

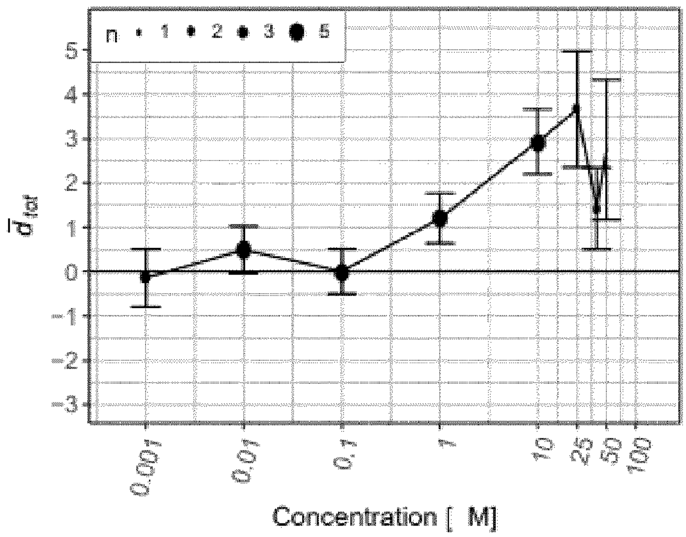


Figure 6

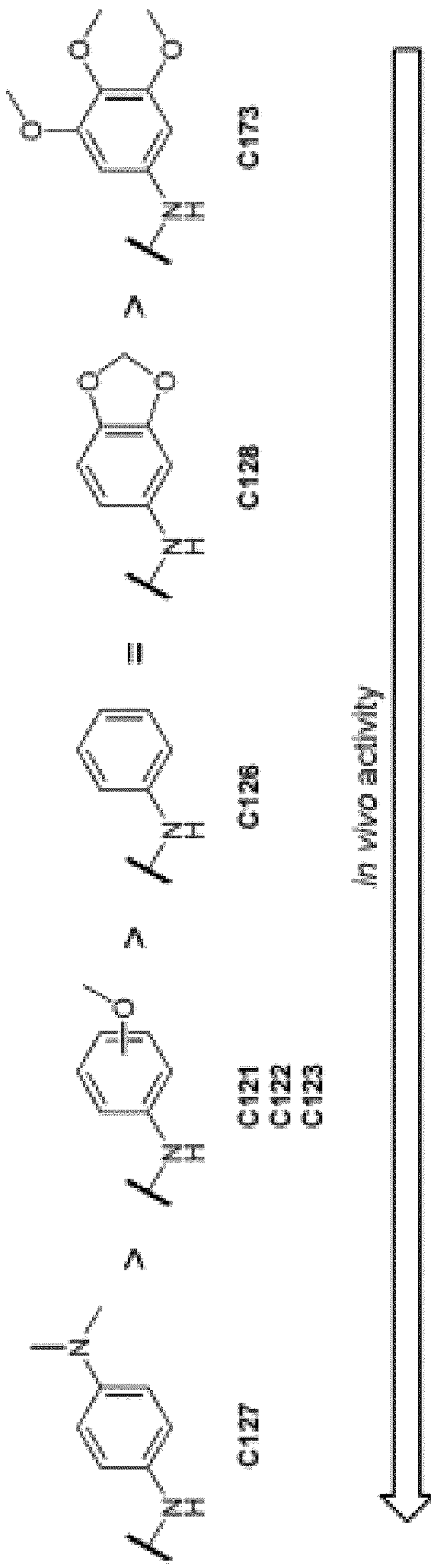


Figure 7

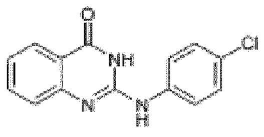

C124

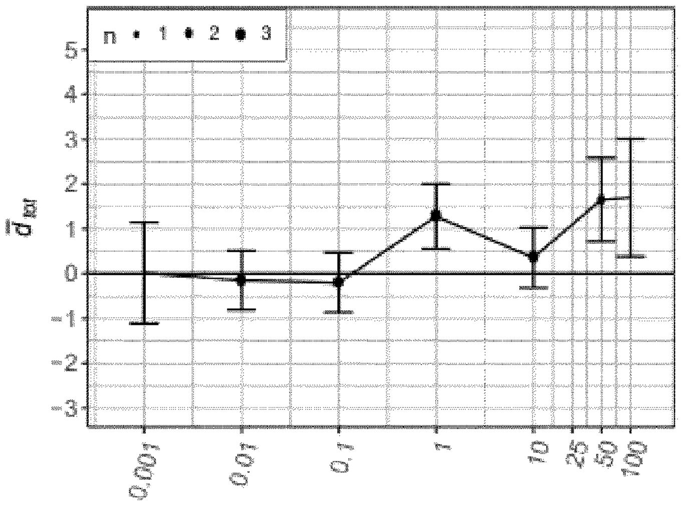


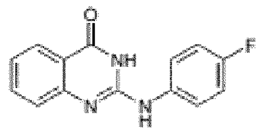

C125

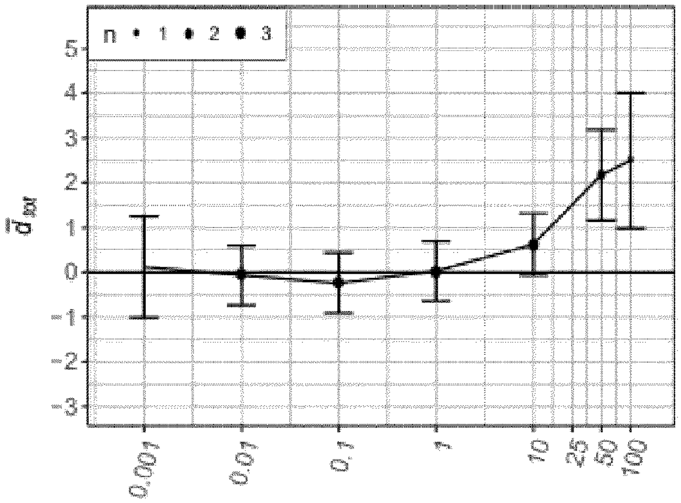


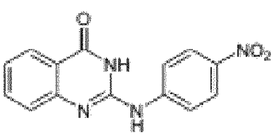

C129

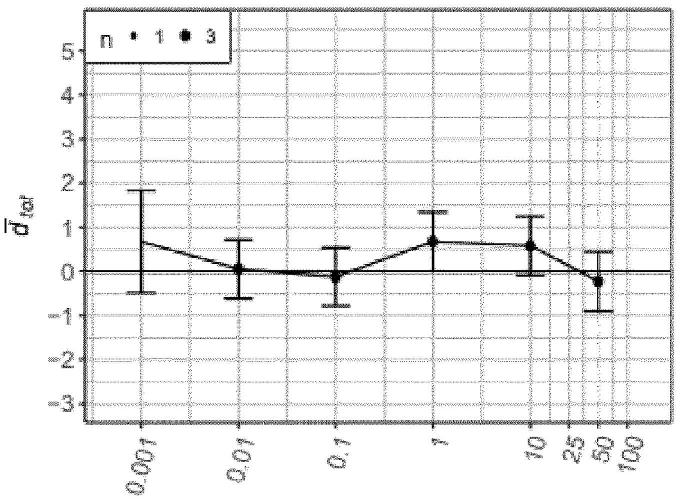


Figure 8

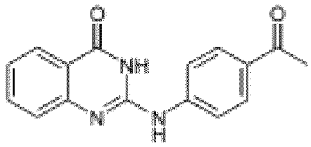

C178

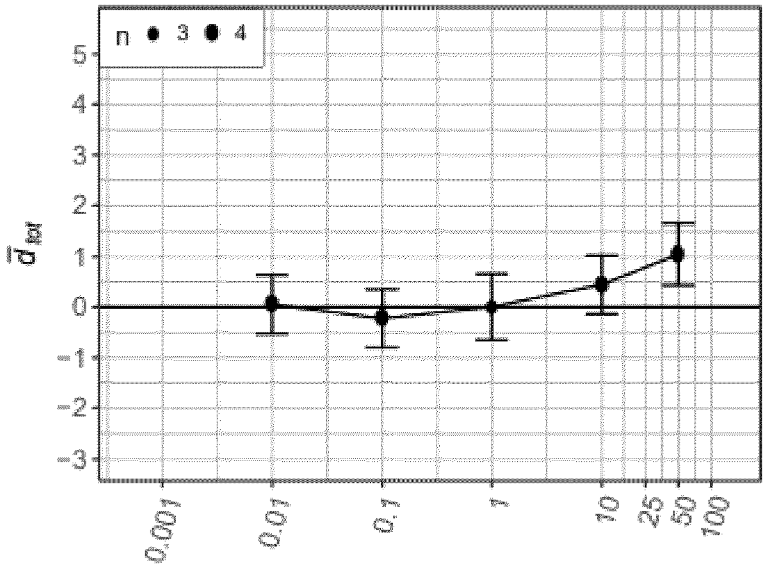


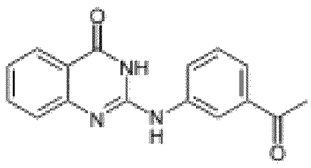

C179

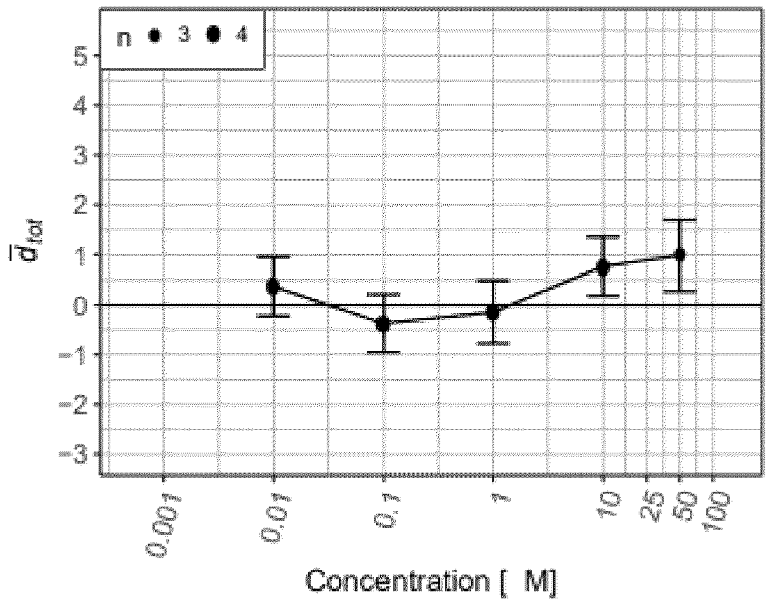


Figure 9

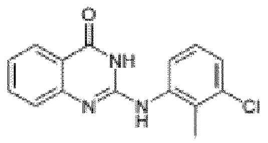

C130

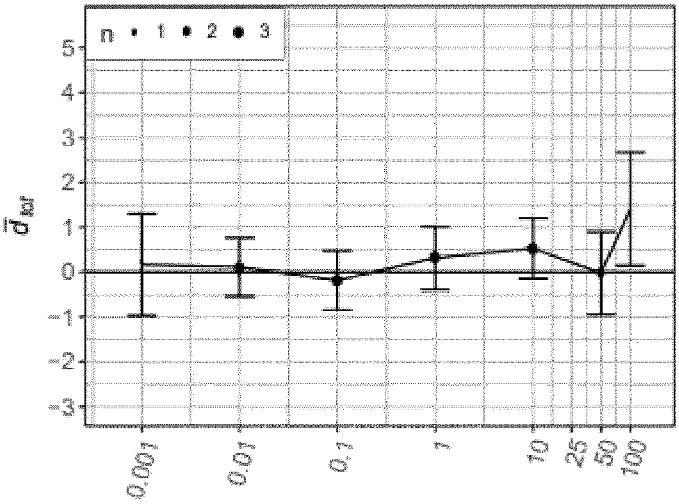


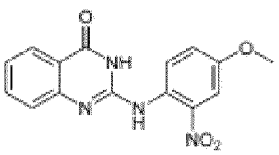

C174

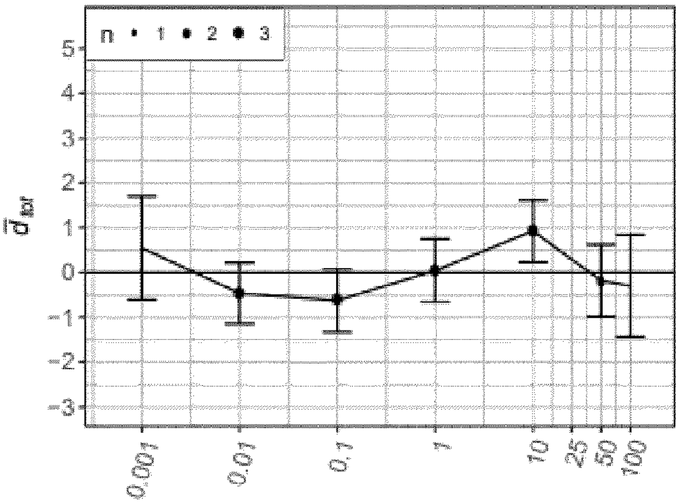


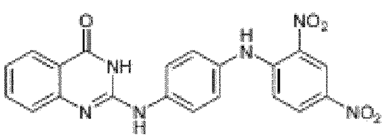

C172

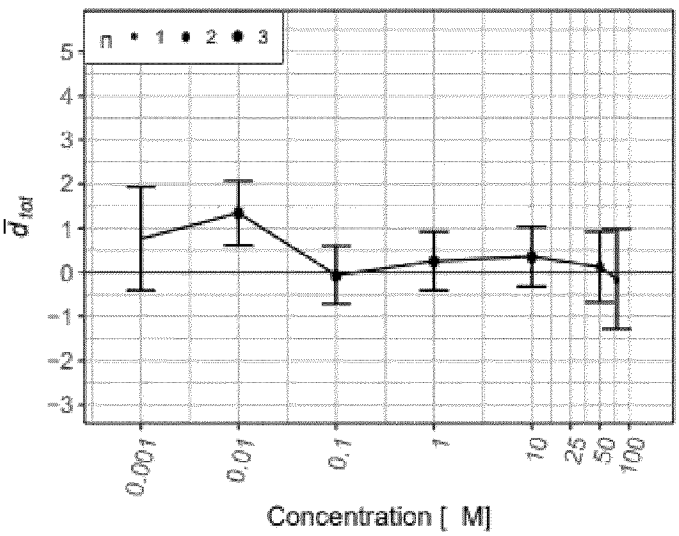


Figure 10

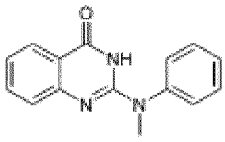

C175

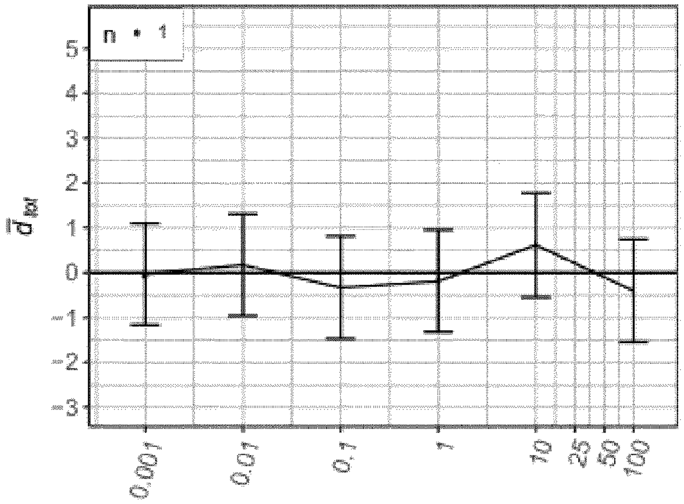


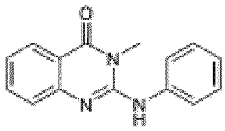

C176

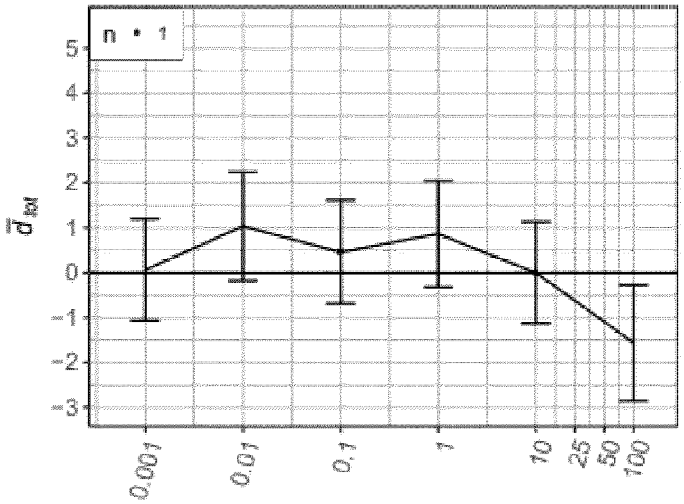


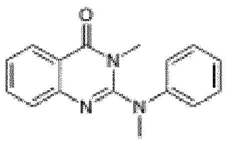

C177

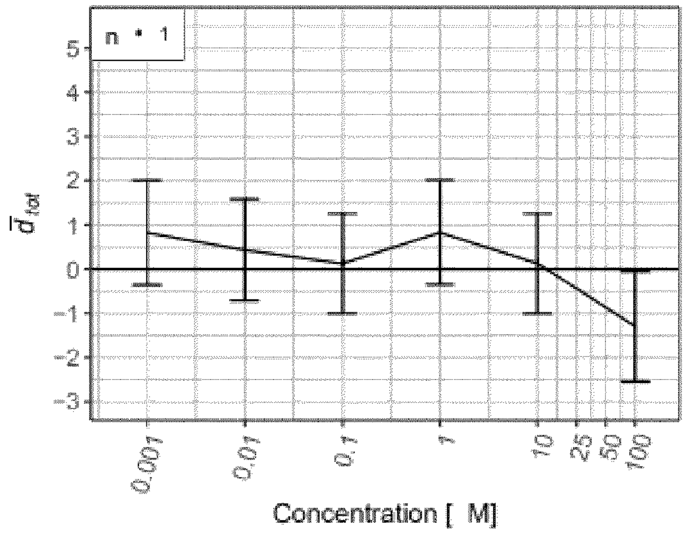


Figure 11

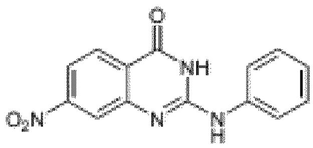

C180

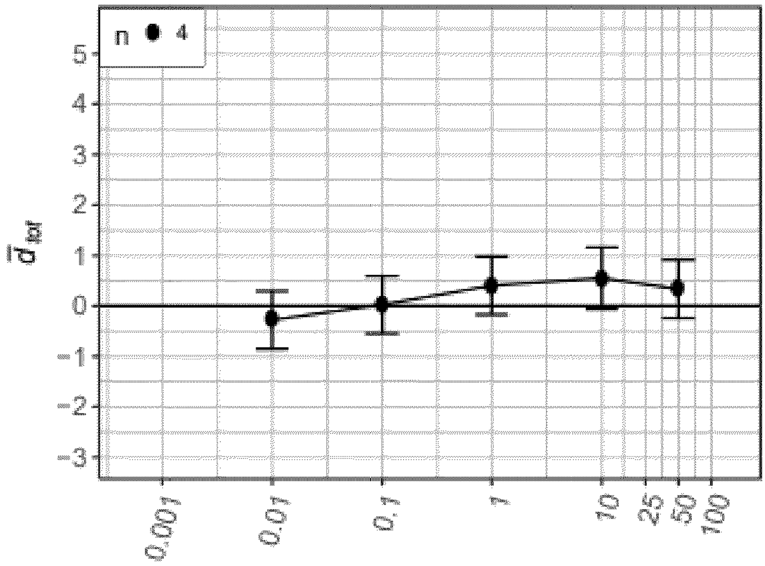


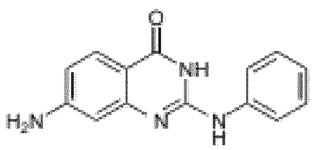

C182

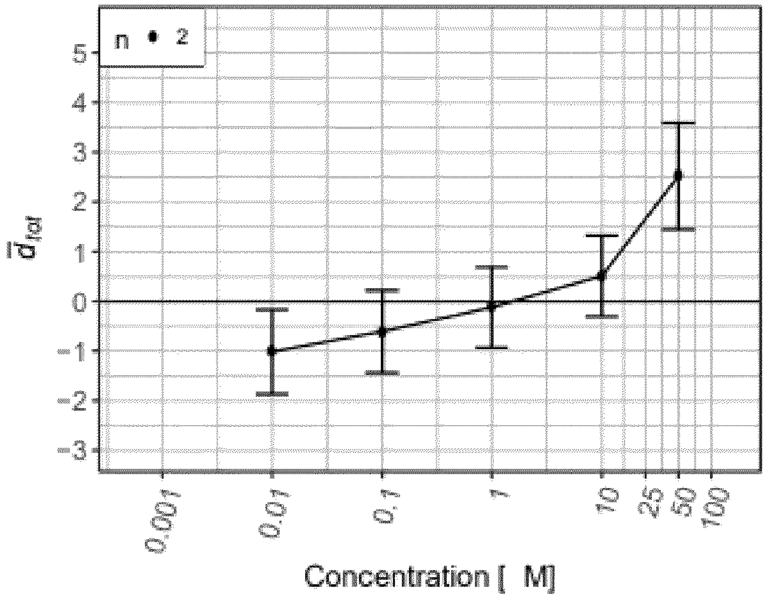


Figure 12

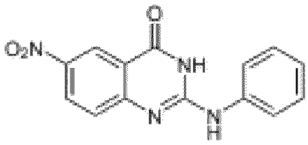

C181

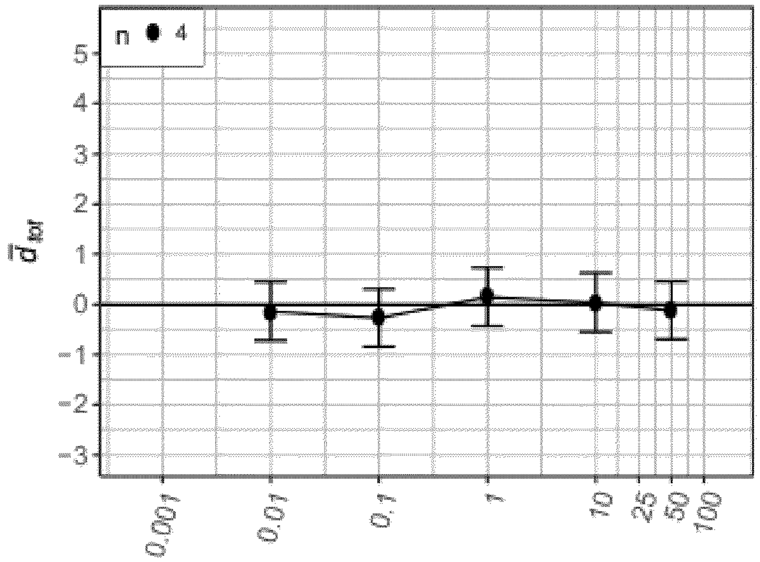


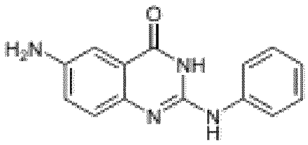

C183

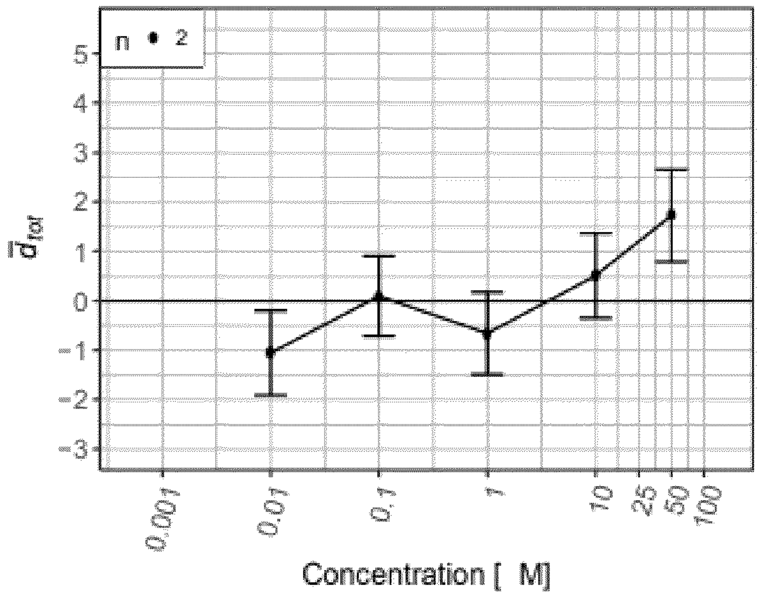


Figure 13

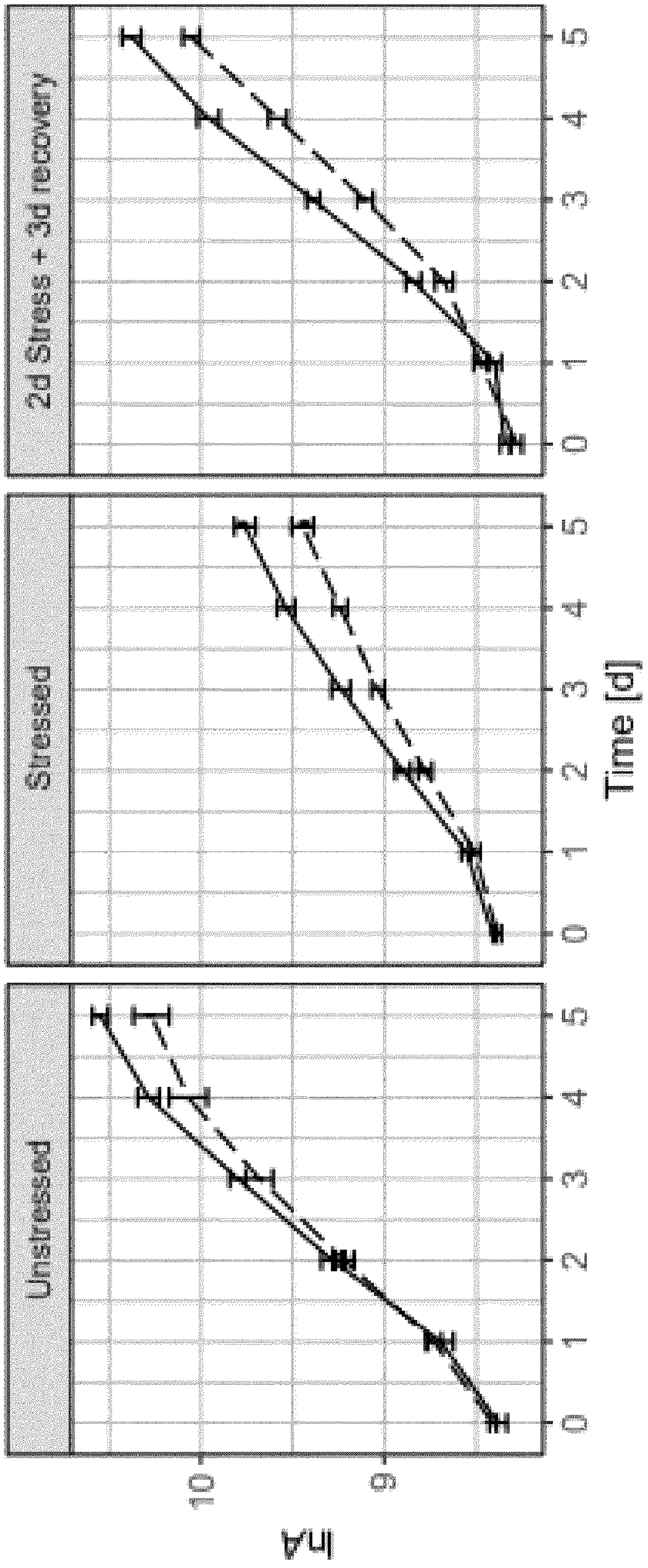
Unstressed
Stressed
2d Stress + 3d recovery
lnA
10
9
0
1
2
3
4
5
Time [d]

Figure 14

Europäisches Patentamt
European Patent Office
Office européen des brevets

# EUROPEAN SEARCH REPORT

Application Number
EP 24 19 0327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2015/173359 A1 (FRACKENPOHL JENS [DE] ET AL) 25 June 2015 (2015-06-25)<br>* paragraphs [0002], [0955], [0956]; claims 1, 3; table 1 *<br>----- | 2,4-6, 11,12<br>7-9 | INV.<br>A01N43/54<br>A01N43/84<br>A01N47/44<br>A01P21/00<br>C07D239/95<br>C07F7/02 |
| X | GB 822 069 A (GEIGY AG J R)<br>21 October 1959 (1959-10-21)<br>* page 1 *<br>* 2,4-bis-ethylamino quinazoline; page 2, line 108 *<br>* page 2, line 108 *<br>----- | 1,3,10 | |
| X | DATABASE REGISTRY [Online]<br>Chemical Abstract Service, Columbus, Ohio, US;<br>26 March 2018 (2018-03-26),<br>XP093228410,<br>Database accession no. 2198858-89-6<br>* the whole document *<br>----- | 13 | |
| | | | TECHNICAL FIELDS SEARCHED (IPC) |
| | | | A01N<br>A01P<br>C07D<br>C07F |

~~The present search report has been drawn up for all claims~~

1

| Place of search | Date of completion of the search | Examiner |
|---|---|---|
| Munich | 27 November 2024 | Beligny, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 24 19 0327

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

3, 8, 13(completely); 1, 2, 4-7, 9-12(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 24 19 0327

The Search Division considers that the present European patent application does not comply with the requirements of unity of invention and relates to several inventions or groups of inventions, namely:

1. claims: 3, 8, 13(completely); 1, 2, 4-7, 9-12(partially)

   Quinazolines of Formula (I) useful as phytoeffector, wherein X is NR4

---

2. claims: 1, 2, 4-7, 9-12(all partially)

   Quinazolines of Formula (I) useful as phytoeffector, wherein X is S, SO, or SO2

---

3. claims: 1, 2, 4-6, 10-12(all partially)

   Quinazolines of Formula (I) useful as phytoeffector, wherein X is O

---

4. claims: 1, 2, 4-6, 10-12(all partially)

   Quinazolines of Formula (I) useful as phytoeffector, wherein X is CH2

---

# ANNEX TO THE EUROPEAN SEARCH REPORT ON EUROPEAN PATENT APPLICATION NO. EP 24 19 0327

This annex lists the patent family members relating to the patent documents cited in the above-mentioned European search report. The members are as contained in the European Patent Office EDP file on
The European Patent Office is in no way liable for these particulars which are merely given for the purpose of information.

27-11-2024

| Patent document cited in search report | Publication date | Patent family member(s) | Publication date |
|---|---|---|---|
| US 2015173359 A1 | 25-06-2015 | AU 2011298423 A1 | 21-03-2013 |
| | | BR 112013005223 A2 | 03-05-2016 |
| | | CN 103228141 A | 31-07-2013 |
| | | EP 2611300 A1 | 10-07-2013 |
| | | ES 2587657 T3 | 26-10-2016 |
| | | PL 2611300 T3 | 31-10-2016 |
| | | RU 2013114710 A | 10-10-2014 |
| | | US 2012157306 A1 | 21-06-2012 |
| | | US 2015173359 A1 | 25-06-2015 |
| | | WO 2012028578 A1 | 08-03-2012 |
| GB 822069 A | 21-10-1959 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **THORSTEN GEIßLER** ; **LUDGER A. WESSJOHANN**. A Whole-Plant Microtiter Plate Assay for Drought Stress Tolerance-Inducing Effect. *Journal of Plant Growth Regulation*, 2011, vol. 30 (4), 504-511